# EUROPEAN PATENT APPLICATION

(11) **EP 4 712 012 A1**
(43) Date of publication of application: **18.03.2026**
(21) Application number: 24911858.9
(22) Date of filing: 29.08.2024
(51) Int. Cl.: G06Q 50/10

(54) **INFORMATION PROCESSING DEVICE, INFORMATION PROCESSING METHOD, AND RECORDING MEDIUM**

(30) Priority: 27.12.2023 JP 2023220753
(71) Applicant: Exevita, Inc., Kamakura-shi, Kanagawa 248-0007 (JP)
(72) Inventor: TADA, Hiroshi, Kamakura-shi, Kanagawa 248-0007 (JP); ANZAI, Yohei, Kamakura-shi, Kanagawa 248-0007 (JP)
(74) Representative: Betten & Resch
(86) International application number: PCT/JP2024/030876
(87) International publication number: WO 2025/141964

(57) **Abstract**

Conventional techniques have not been able to present a user's keystone habit. The user's keystone habit can be presented by using an information processing apparatus including: a set determination unit configured to reference an action management unit in which pieces of action information specifying a user's actions are stored in association with four or more time slots, to acquire the number of occurrences of an action set that is a series of two or more pieces of action information that are temporally continuous, and to determine an action set whose number of occurrences satisfies a set condition on the number of occurrences of the action set; a keystone habit acquisition unit configured to acquire keystone habit information including a piece of action information that corresponds to an initial time slot among the series of two or more pieces of action information in the action set determined by the set determination unit and serves as a starting point; and a keystone habit output unit configured to output the keystone habit information.

## Description

### Technical field

The present invention relates to an information processing apparatus and the like that acquire and output a keystone habit serving as a starting point of a user's action set.

### Background Art

Conventionally, there has been an action determination system that determines the sleep and other actions of a resident using energy consumption data (see Patent Document 1).

### Citation List

### Patent Document

Patent Document 1: JP 6470497B

### Summary of Invention

### Technical Problem

However, in the conventional technology, it is not possible to present the user's keystone habit. Note that a keystone habit refers to a key habit. In this specification, a "habit" is what is indicated by action information.

### Solution to Problem

An information processing apparatus according to a first aspect of the present invention is an information processing apparatus including: a set determination unit configured to reference an action management unit in which pieces of action information specifying a user's actions are stored in association with four or more time slots, and to determine an action set that is a series of two or more actions and satisfies a set condition; a keystone habit acquisition unit configured to acquire keystone habit information including a piece of action information specifying an action serving as a starting point of the action set determined by the set determination unit; and a keystone habit output unit configured to output the keystone habit information.

With such a configuration, the user's keystone habit can be presented.

An information processing apparatus according to a second aspect of the present invention is an information processing apparatus according to the first aspect of the invention, wherein the keystone habit acquisition unit acquires keystone habit information that includes a piece of action information specifying an action serving as a starting point of the action set and a start time included in a time slot corresponding to the piece of action information.

With such a configuration, the user's keystone habit and the start time thereof and the like can be presented.

An information processing apparatus according to a third aspect of the present invention is an information processing apparatus according to the first or the second aspect of the invention, further including: a time acquisition unit configured to acquire a time; a position acquisition unit configured to acquire position information corresponding to the time; and an action estimation unit configured to, using two or more pieces of action source information including the position information corresponding to the time, acquire a piece of action information specifying an action of the user during a time slot specified by the time contained in the two or more pieces of action source information, wherein at least one of the two or more pieces of action information corresponding to time slots in the action management unit is the piece of action information corresponding to the time slot acquired by the action estimation unit.

With such a configuration, the user's action can be estimated by using position information corresponding to the time.

An information processing apparatus according to a fourth aspect of the present invention is an information processing apparatus according to the first or the second aspect of the invention, wherein the pieces of action information in the action management unit are associated with time slots and pieces of emotion information specifying emotions, and the set determination unit uses the pieces of emotion information to determine the action set.

With such a configuration, the action set can be determined by using emotion information.

An information processing apparatus according to a fifth aspect of the present invention is an information processing apparatus according to any one of the first to the fourth aspect of the invention, further including: a query acceptance unit configured to accept a query including a time and a piece of action information, wherein the keystone habit acquisition unit determines a piece of action information specifying an action serving as a starting point of the action set determined by the set determination unit, and a time slot corresponding to the time and the piece of action information contained in the query, and acquires keystone habit information including a piece of action information specifying an action serving as a starting point of an action set corresponding to a time slot that precedes the determined time slot and is closest to the determined time slot.

With such a configuration, a keystone habit corresponding to a query from the user can be presented to the user.

An information processing apparatus according to a sixth aspect of the present invention is an information processing apparatus according to any one of the first to the fourth aspect of the invention, wherein the keystone habit output unit outputs a recommendation including the keystone habit information.

With such a configuration, a recommendation using the keystone habit can be made.

An information processing apparatus according to a seventh aspect of the present invention is an information processing apparatus according to a fifth aspect of the invention, wherein the pieces of action information in the action management unit are associated with pieces of emotion information specifying emotions, the information processing apparatus further includes a recommendation acquisition unit configured to acquire a piece of emotion information corresponding to the piece of action information included in the keystone habit information or the piece of action information contained in the query, and to form a recommendation using the piece of emotion information and the keystone habit information, and the keystone habit output unit outputs the recommendation.

With such a configuration, a recommendation using the keystone habit and the emotion information can be made.

### Advantageous Effects of Invention

With an information processing apparatus according to the present invention, the user's keystone habit can be presented.

### Brief Description of Drawings

FIG. 1 is a conceptual diagram of an information system A including a location information production apparatus 1 according to a first embodiment.
FIG. 2 is a block diagram of the location information production apparatus 1 according to the same.
FIG. 3 is a flowchart illustrating an example of operation of the location information production apparatus 1 according to the same.
FIG. 4 is a flowchart illustrating an example of time-series intensity acquisition processing according to the same.
FIG. 5 is a flowchart illustrating an example of fixed information acquisition processing according to the same.
FIG. 6 is a flowchart illustrating an example of type judgment processing according to the same.
FIG. 7 is a diagram showing a time-series radio wave intensity management table according to the same.
FIG. 8 is a diagram showing a location information management table according to the same.
FIG. 9 is a conceptual diagram of an information system C according to a second embodiment.
FIG. 10 is a block diagram of a terminal apparatus 2 according to the same.
FIG. 11 is a flowchart illustrating a first operation example of the terminal apparatus 2 according to the same.
FIG. 12 is a flowchart illustrating an example of movement judgment processing according to the same.
FIG. 13 is a flowchart illustrating an example of position estimation processing according to the same.
FIG. 14 is a flowchart illustrating a second operation example of the terminal apparatus 2 according to the same.
FIG. 15 is a flowchart illustrating a second example of type judgment processing according to the same.
FIG. 16 is a conceptual diagram of an information system D according to a third embodiment.
FIG. 17 is a block diagram of the information system D according to the same.
FIG. 18 is a block diagram of an action acquisition apparatus 3 according to the same.
FIG. 19 is a flowchart illustrating an example of operation of the action acquisition apparatus 3 according to the same.
FIG. 20 is a flowchart illustrating an example of action source acquisition processing according to the same.
FIG. 21 is a flowchart illustrating an example of position estimation processing according to the same.
FIG. 22 is a flowchart illustrating an example of first action estimation processing according to the same.
FIG. 23 is a flowchart illustrating an example of second action estimation processing according to the same.
FIG. 24 is a flowchart illustrating an example of third action estimation processing according to the same.
FIG. 25 is a flowchart illustrating an example of first emotion estimation processing according to the same.
FIG. 26 is a flowchart illustrating an example of second emotion estimation processing according to the same.
FIG. 27 is a flowchart illustrating an example of third emotion estimation processing according to the same.
FIG. 28 is a flowchart illustrating an example of output formation processing according to the same.
FIG. 29 is a flowchart illustrating an example of first action learning processing according to the same.
FIG. 30 is a flowchart illustrating an example of second action learning processing according to the same.
FIG. 31 is a flowchart illustrating an example of first emotion learning processing according to the same.
FIG. 32 is a flowchart illustrating an example of second emotion learning processing according to the same.
FIG. 33 is a diagram showing an action source management table according to the same.
FIG. 34 is a diagram showing an action and emotion management table according to the same.
FIG. 35 is a diagram showing a time slot information management table according to the same.
FIG. 36 is an example of an output according to the same.
FIG. 37 is a conceptual diagram of an information system E according to a fourth embodiment.
FIG. 38 is a block diagram of the information system E according to the same.
FIG. 39 is a block diagram of an action acquisition apparatus 5 according to the same.
FIG. 40 is a flowchart illustrating an example of operation of the action acquisition apparatus 5 according to the same.
FIG. 41 is a flowchart illustrating an example of operation of a terminal apparatus 6 according to the same.
FIG. 42 is a conceptual diagram of an information system F according to a fifth embodiment.
FIG. 43 is a block diagram of the information system F according to the same.
FIG. 44 is a flowchart illustrating an operation example of an information processing apparatus 7 according to the same.
FIG. 45 is a flowchart illustrating keystone habit acquisition processing according to the same.
FIG. 46 is a flowchart illustrating an example of recommendation acquisition processing according to the same.
FIG. 47 is a diagram showing a keystone habit management table according to the same.
FIG. 48 is a diagram showing an example of output according to the same.
FIG. 49 is a conceptual diagram of an information system G according to a sixth embodiment.
FIG. 50 is a block diagram of the information system G according to the same.
FIG. 51 is a block diagram of a computer system according to the above embodiments.

### Description of Embodiments

Hereinafter, embodiments of the information processing apparatus and the like will be described with reference to the drawings. It should be noted that in the embodiments, constituent elements with the same reference signs perform similar operations, and therefore, repeated descriptions thereof may be omitted.

### First Embodiment

The present embodiment describes a location information production apparatus. The location information production apparatus is an apparatus that acquires location information, which will be described later, of a specific location.

Note that in the present description, the fact that information X is associated with information Y means that information Y can be acquired from information X, or that information X can be acquired from information Y, and the method for association is not limited. For example, information X and information Y may be linked or exist in the same buffer, information X may be included in information Y, or information Y may be included in information X.

In addition, in this specification, selecting or determining information Z means, for example, acquiring information Z, acquiring a pointer to information Z, acquiring the ID of information Z, setting a flag for information Z, and it is only required that information Z be accessible.

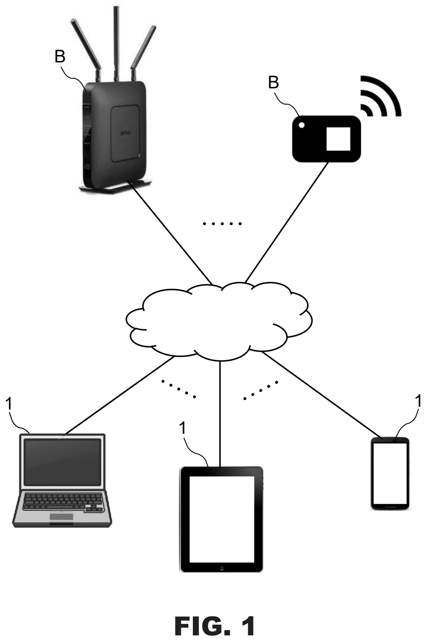

FIG. 1 is a conceptual diagram for an information system A that includes a location information production apparatus 1 according to the present embodiment. The information system A includes the location information production apparatus 1 and three or more communication apparatuses B.

Each of the three or more communication apparatuses B is an apparatus that transmits radio waves to another apparatus such as the location information production apparatus 1. The communication apparatus B transmits an apparatus identifier that identifies the communication apparatus B to another apparatus. The communication apparatus B may be, for example, a Wi-fi router or a communication apparatus using BLE (Bluetooth Low Energy), but there is no limitation.

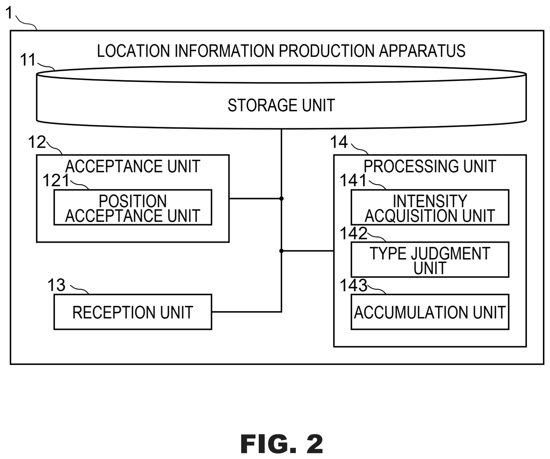

FIG. 2 is a block diagram for the location information production apparatus 1 according to the present embodiment. The location information production apparatus 1 includes a storage unit 11, an acceptance unit 12, a reception unit 13, and a processing unit 14. The acceptance unit 12 includes a position acceptance unit 121. The processing unit 14 includes an intensity acquisition unit 141, a type judgment unit 142, and an accumulation unit 143.

The acceptance unit 12 accepts various instructions and information. Examples of the various instructions and information include position information, which will be described later. Any input means, such as a touch panel, a keyboard, a mouse, a menu screen, or the like, may be employed to input various instructions and information.

The position acceptance unit 121 accepts position information regarding a specific location. The position acceptance unit 121 typically accepts position information regarding each of three or more specific locations. For example, the position acceptance unit 121 accepts position information input from a user. For example, the position acceptance unit 121 reads out position information from the storage unit 11.

The specific location is a specific indoor location, but may also be a specific outdoor location. Here, the position information is information that specifies an indoor or outdoor position. For example, the position information is three-dimensional coordinate values (x,y,z) indicating a relative indoor or outdoor position, but may also be two-dimensional coordinate values (x,y). Note that there is no limitation on the origin of the coordinate values that specify a relative indoor or outdoor position. It is preferable that the specific outdoor location is a location where GPS signals are difficult to reach, such as among high-rise buildings or in a forest, but there is no limitation thereon. The position information may be information (for example, a character string) or an ID that allows a person to recognize a location. Such position information may be, for example, labels such as "living room," "work room," "conference room," "east side of the library," "toy section of the department store," and the like.

The position acceptance unit 121 may generate a unique ID. Such a unique ID may be considered as a label and position information.

The position acceptance unit 121 does not have to receive position information. In such a case, the position acceptance unit 121 is unnecessary.

The reception unit 13 receives radio waves containing apparatus identifiers from three or more communication apparatuses B at a specific location. The reception unit 13 typically successively receives radio waves containing apparatus identifiers from three or more communication apparatuses B.

The apparatus identifiers are pieces of information that respectively identify the communication apparatuses B. The apparatus identifiers are, for example, the respective IDs of the communication apparatuses B or the respective names of the communication apparatuses B. The reception of radio waves may be considered as the reception of information.

The processing unit 14 performs various kinds of processing. The various kinds of processing are, for example, the processing performed by the intensity acquisition unit 141, the type judgment unit 142, and the accumulation unit 143.

The intensity acquisition unit 141 acquires, for each of the three or more communication apparatuses B, the intensity of the radio wave received from the communication apparatus B. The intensity acquisition unit 141 acquires the radio wave intensity in a pair with the apparatus identifier of the communication apparatus B. The intensity acquisition unit 141 acquires time-series radio wave intensities. "Time-series radio wave intensities" means two or more radio wave intensities that are consecutive in time. It is understood that "consecutive in time" means that there may be time intervals.

The type judgment unit 142 judges whether or not each of the three or more communication apparatuses B is a fixed terminal or a mobile terminal, using the time-series radio wave intensities acquired by the intensity acquisition unit 141. The fixed terminal is a communication apparatus whose installation position is fixed. The mobile terminal is a communication apparatus whose installation position is not fixed and that is moving.

For example, the type judgment unit 142 acquires the degree of variation of two or more radio wave intensities that are paired with one apparatus identifier and are consecutive in a time series, and if the degree of variation is not less than a threshold value or greater than a threshold value, the type judgment unit 142 judges that the communication apparatus B identified by the one apparatus identifier is a mobile terminal. For example, the type judgment unit 142 acquires the degree of variation of two or more time-series radio wave intensities that are paired with one apparatus identifier and are consecutive in time, and if the degree of variation is not greater than a threshold value or less than a threshold value, the type judgment unit 142 judges that the communication apparatus B identified by the one apparatus identifier is a fixed terminal.

Note that the degree of variation is information indicating the degree of variation or change in the time-series radio wave intensities. The degree of variation is, for example, a variance, a standard deviation, or a number based on a difference (for example, a difference, or a value obtained by adding the differences between any two radio wave intensities among the three or more radio wave intensities that are consecutive in time).

For example, if a number that is the number of radio wave intensities acquired in a predetermined period of time and that is the number of time-series radio wave intensities consecutive in time and paired with one apparatus identifier, is not greater than a threshold value or less than a threshold value, the type judgment unit 142 judges that the communication apparatus B identified by the one apparatus identifier is a mobile terminal.

The accumulation unit 143 forms and accumulates location information that contains the apparatus identifier and the radio wave intensity of the communication apparatuses B judged to be a fixed terminal by the type judgment unit 142. It is preferable that the accumulation unit 143 forms and accumulates location information that contains an apparatus identifier and a radio wave intensity for each of the three or more communication apparatuses B.

The accumulation unit 143 forms and accumulates location information that contains the apparatus identifier and the radio wave intensity of the communication apparatus B judged to be a fixed terminal by the type judgment unit 142. It is preferable that the accumulation unit 143 forms and accumulates location information that contains an apparatus identifier, a radio wave intensity, and position information regarding a specific location for each of the three or more communication apparatuses B. The accumulation unit 143 accumulates location information in the storage unit 11, for example, but may accumulate it in another apparatus. It is preferable that location information contains position information, but it does not have to contain position information. Location information may only contain an apparatus identifier and a radio wave intensity.

The radio wave intensity accumulated in the accumulation unit 143 is typically a representative value of the time-series radio wave intensities of the radio waves from the communication apparatus B. The representative value is, for example, the median, average, maximum value, or minimum value.

It is preferable that the storage unit 11 is realized using a non-volatile recording medium, but it can be realized using a volatile recording medium.

There is no limitation on the process in which information is stored in the storage unit 11. For example, information may be stored in the storage unit 11 via a recording medium, or information transmitted via a communication line or the like may be stored in the storage unit 11, or information input via an input device may be stored in the storage unit 11.

The acceptance unit 12 and the position acceptance unit 121 can be realized using a device driver for the input means such as a touch panel or a keyboard, or control software or the like for controlling the menu screen.

The reception unit 13 is typically realized using a wireless or wired communication means.

The processing unit 14, the intensity acquisition unit 141, the type judgment unit 142, and the accumulation unit 143 can typically be realized using a processor, a memory, or the like. The processing procedures performed by the processing unit 14 and so on are typically realized using software, and the software is recorded on a recording medium such as a ROM. However, such processing procedures may be realized using hardware (a dedicated circuit). Note that the processor may be a CPU, an MPU, a GPU, or the like, and there is no limitation on the type thereof.

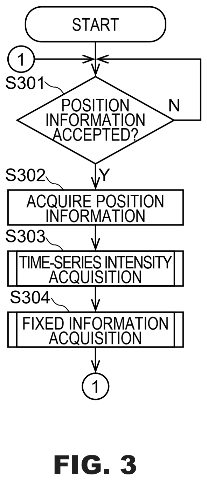

Next, examples of operations of the location information production apparatus 1 will be described with reference to the flowchart in FIG. 3.

(Step S301) The position acceptance unit 121 judges whether or not position information regarding a specific location has been accepted. If position information has been accepted, processing proceeds to step S302, and if position information has not been accepted, processing returns to step S301.

(Step S302) The accumulation unit 143 acquires the position information accepted in step S301.

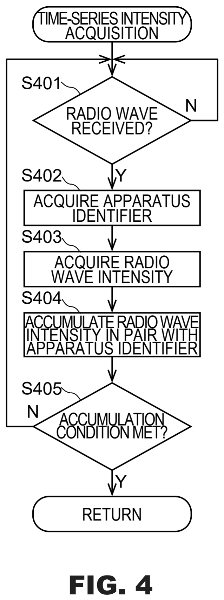

(Step S303) The processing unit 14 and so on perform time-series intensity acquisition processing. Time-series intensity acquisition processing is processing performed to acquire time-series radio wave intensities of radio waves from each of three or more communication apparatuses B. An example of time-series intensity acquisition processing will be described with reference to the flowchart in FIG. 4.

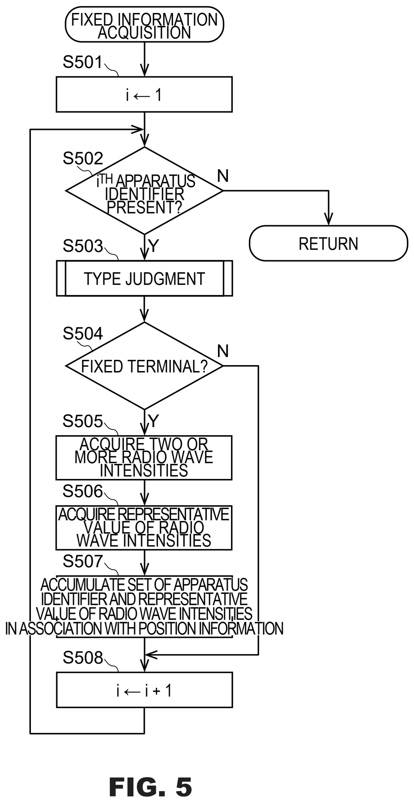

(Step S304) The processing unit 14 and so on perform fixed information acquisition processing. Processing returns to step S301. Fixed information acquisition processing is processing performed to acquire a radio wave intensity from a fixed terminal at the location specified by the position information accepted in step S301. An example of fixed information acquisition processing will be described with reference to the flowchart in FIG. 5.

It is preferable that, in the flowchart in FIG. 3, the user holding the location information production apparatus 1 moves to each of the three or more specific locations, and the location information production apparatus 1 accepts location information for each of the three or more specific locations, and repeatedly performs the processing in S301 to S304.

In the flowchart shown in FIG. 3, processing is terminated when power is turned off or an interruption is made to terminate the processing.

Next, an example of the time-series intensity acquisition processing in step S303 will be described with reference to the flowchart in FIG. 4.

(Step S401) The reception unit 13 judges whether or not a radio wave has been received from any of the communication apparatuses B. If a radio wave has been received, processing proceeds to step S402, and otherwise processing returns to step S401.

(Step S402) The accumulation unit 143 acquires an apparatus identifier corresponding to the radio wave received in step S401.

(Step S403) The intensity acquisition unit 141 acquires the intensity of the radio wave received in step S401.

(Step S404) The accumulation unit 143 accumulates the radio wave intensity acquired in step S403 in a buffer (not shown) in association with the apparatus identifier acquired in step S402.

(Step S405) The accumulation unit 143 judges whether or not a location information accumulation condition is met. If the accumulation condition is met, processing returns to high level processing, and otherwise processing returns to step S401. The accumulation condition is, for example, that a threshold time or more has elapsed from the reception of the location information regarding the specific location, that a threshold number or more of radio wave intensities paired with three or more apparatus identifiers have been accumulated, or the like.

Next, an example of the fixed information acquisition processing in step S304 will be described with reference to the flowchart in FIG. 5.

(Step S501) The type judgment unit 142 substitutes 1 for a counter i.

(Step S502) The type judgment unit 142 judges whether or not an i^{th} apparatus identifier is present in a buffer (not shown). If the i^{th} apparatus identifier is present, processing proceeds to step S503, and if the i^{th} apparatus identifier is not present, processing returns to higher level processing.

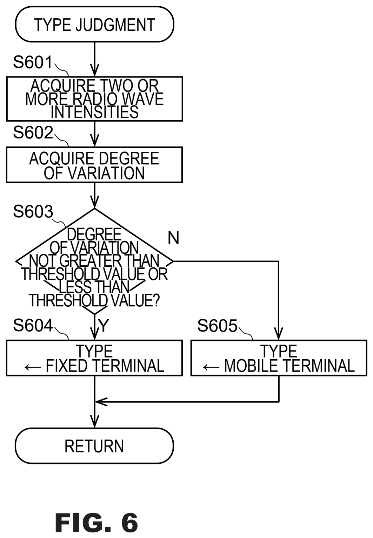

(Step S503) The type judgment unit 142 judges the type of the communication apparatuses B identified by the i^{th} apparatus identifier. An example of such type judgment processing will be described with reference to the flowchart in FIG. 6.

(Step S504) If the result of the judgment in step S503 is "fixed terminal", processing proceeds to step S505, and if the result of the judgment is "mobile terminal", the processing proceeds to step S508.

(Step S505) The accumulation unit 143 acquires two or more radio wave intensities paired with the i^{th} apparatus identifier from a buffer (not shown).

(Step S506) The accumulation unit 143 acquires a representative value of the two or more radio wave intensities.

(Step S507) The accumulation unit 143 accumulates the set of the i^{th} apparatus identifier and the representative value of the radio wave intensities acquired in step S506 in the storage unit 11 in association with the position information accepted in step S301.

(Step S508) The type judgment unit 142 increments the counter i by 1. Processing returns to step S502.

Note that, in the flowchart in FIG. 5, the accumulation unit 143 may newly acquire the latest radio wave intensity in step S506 instead of the representative value of the two or more radio wave intensities.

Next, an example of the type judgment processing in step S503 will be described with reference to the flowchart in FIG. 6.

(Step S601) The type judgment unit 142 acquires the two or more radio wave intensities paired with the i^{th} apparatus identifier in step S502 from a buffer (not shown).

(Step S602) The type judgment unit 142 acquires the degree of variation in the two or more radio wave intensities acquired in step S601.

(Step S603) The type judgment unit 142 judges whether or not the degree of variation acquired in step S602 is not greater than a threshold value or less than a threshold value. If the degree of variation is not greater than the threshold value or less than the threshold value, processing proceeds to step S604, and if the degree of variation is not less than the threshold value or greater than the threshold value, processing proceeds to step S605.

(Step S604) The type judgment unit 142 judges that the type of the communication apparatus B is "fixed terminal". Processing returns to higher level processing.

(Step S605) The type judgment unit 142 judges that the type of the communication apparatus B is "mobile terminal". Processing returns to higher level processing.

Hereinafter, examples of specific operations of the location information production apparatus 1 according to the present embodiment will be described. Here, it is assumed that the accumulation condition is that a predetermined time (for example, three minutes) has elapsed since the location information regarding the specific location was accepted.

It is assumed that, for example, a user Ais in a certain place indoors (for example, the user A's home or a department store that the user A often visits). It is assumed that the user A thereafter inputs position information (x₁,y₁) to the location information production apparatus 1.

Next, the position acceptance unit 121 of the location information production apparatus 1 accepts the position information (x₁,y₁) regarding the specific location. Next, the accumulation unit 143 acquires the accepted position information (x₁,y₁) into a buffer (not shown).

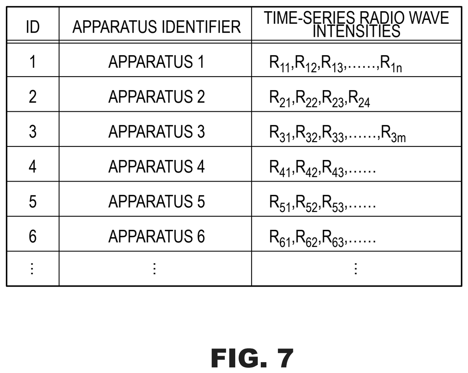

Thereafter, the reception unit 13 receives a radio wave that contains an apparatus identifier from each of three or more communication apparatuses B during a predetermined period of time (for example, three minutes). Thereafter, the accumulation unit 143 acquires the apparatus identifier contained in the received radio wave. In addition, the intensity acquisition unit 141 acquires the intensity of the received radio wave. Next, the accumulation unit 143 adds the acquired radio wave intensity to a buffer (not shown) in association with the acquired apparatus identifier. As a result, the time series radio wave intensity management table shown in FIG. 7 is formed in the buffer (not shown). The time series radio wave intensity management table shown in FIG. 7 is a table at the specific location indicated by the position information (x₁,y₁).

The time series radio wave intensity management table is a table for managing time-series radio wave intensities for each communication apparatus B. The time series radio wave intensity management table is a table for managing two or more records each containing "ID", "apparatus identifier", and "time-series radio wave intensities." The "ID" is information identifying a record. The "time-series radio wave intensities" are radio wave intensities that are consecutive in time. "R₁₁", "R₁₂", "R₂₁", and so on are radio wave intensities.

After the management table in FIG. 7 is formed, the accumulation unit 143 judges that the accumulation condition for the location information is met because a predetermined period of time (for example, 3 minutes) has elapsed since the position information (x₁,y₁) regarding the specific location was accepted by the position acceptance unit 121.

Next, the type judgment unit 142 acquires the degree of variation in the time-series radio wave intensities for each record in FIG. 7, and judges whether each communication apparatus B is a fixed terminal or a mobile terminal according to the flowchart in FIG. 6. It is assumed that the type judgment unit 142 thereafter judges that the communication apparatuses B identified by the apparatus identifiers "apparatus 1, apparatus 3, apparatus 4, apparatus 6, ..." are fixed terminals, and the communication apparatuses B identified by the apparatus identifiers "apparatus 2, apparatus 5, ..." are mobile terminals.

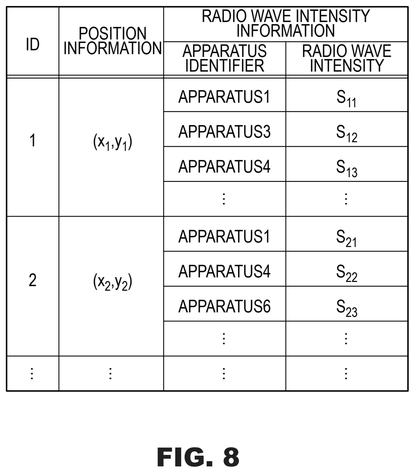

Next, the accumulation unit 143 forms radio wave intensity information by pairing the apparatus identifier of each of the communication apparatuses B that are fixed terminals with the representative value of the radio wave intensities corresponding thereto. Thereafter, the accumulation unit 143 accumulates each of the multiple pieces of radio wave intensity information in association with the position information (x₁,y₁). Through such processing, the record with "ID = 1" in the location information management table in FIG. 8 is formed. Note that the accumulation unit 143 may only accumulate apparatus identifiers and radio wave intensities. In such a case, each record in FIG. 8 does not contain position information. In addition, in such a case, the position acceptance unit 121 does not need to accept position information regarding the specific location.

The location information management table is a table for managing location information. The location information management table stores multiple records associated with pieces of position information and each containing "ID", "apparatus identifier", and "radio wave intensity information". "Radio wave intensity" contains "apparatus identifier" and "radio wave intensity".

Through the above processing, the location information at the specific location 1 indicated by the position information (x₁,y₁) is accumulated.

The user A moves to a specific location 2 at the position indicated by position information (x₂,y₂) while holding the location information production apparatus 1, and performs the same processing as above. As a result, the location information production apparatus 1 forms and accumulates the record with "ID = 2" in the location information management table in FIG. 8. Furthermore, the user A moves to each of one or more specific locations, including a specific location 3 while holding the location information production apparatus 1, and performs the same processing as above. As a result, the location information production apparatus 1 forms and accumulates the records with "ID = 3" and the following IDs (not shown) in the location information management table in FIG. 8.

As described above, according to the present embodiment, it is possible to acquire location information used to acquire the positions of the terminal apparatuses indoors. That is to say, according to the present embodiment, it is possible to produce three or more pieces of location information used to acquire the positions of terminal apparatuses indoors.

Note that the processing in the present embodiment may be realized using software. This software may be distributed through software downloading or the like. Also, this software may be recorded on a recording medium such as a CD-ROM and distributed. Note that the same applies to the other embodiments in the present description. Note that the software that realizes the location information production apparatus 1 according to the present embodiment is the program described below. That is to say, this program is a program that enables a computer to function as: a position acceptance unit configured to accept position information regarding a specific location; a reception unit configured to receive, from each communication apparatus of three or more communication apparatuses, a radio wave containing an apparatus identifier identifying the communication apparatus; an intensity acquisition unit configured to acquire time-series radio wave intensities for each communication apparatus of the three or more communication apparatuses; a type judgment unit configured to judge, for each communication apparatus of the three or more communication apparatuses, whether or not the communication apparatus is a fixed terminal that is a fixed communication apparatus or a mobile terminal that is a moving communication apparatus, using the time-series radio wave intensities acquired by the intensity acquisition unit; and an accumulation unit configured to form and accumulate location information containing apparatus identifiers and radio wave intensities of three or more communication apparatuses judged to be fixed terminals by the type judgment unit and the position information regarding the specific location.

### Second Embodiment

The present embodiment describes a terminal apparatus that receives radio waves from three or more communication apparatuses B, judges the type of each communication apparatus B, using time-series radio wave intensities, and acquires and outputs a terminal position indicating the position of the terminal apparatus indoors, using radio wave intensities from only the communication apparatuses B of the "fixed terminal" type.

The present embodiment also describes a terminal apparatus that judges whether the terminal apparatus is moving or stopped, and acquires and outputs the terminal position using the determination result.

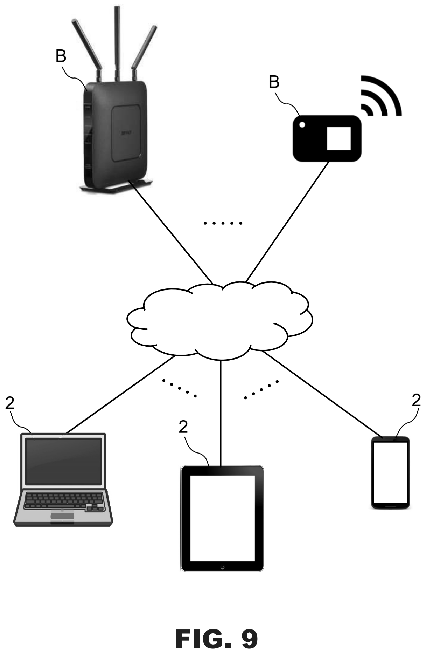

FIG. 9 is a conceptual diagram for an information system C according to the present embodiment. The information system C includes one or more terminal apparatuses 2 and three or more communication apparatuses B.

The terminal apparatuses 2 are terminals that can acquire indoor position information. Each terminal apparatus 2 is, for example, a smart phone, a tablet terminal, a smart watch, a so-called personal computer, or the like, and there is no limitation on the type thereof.

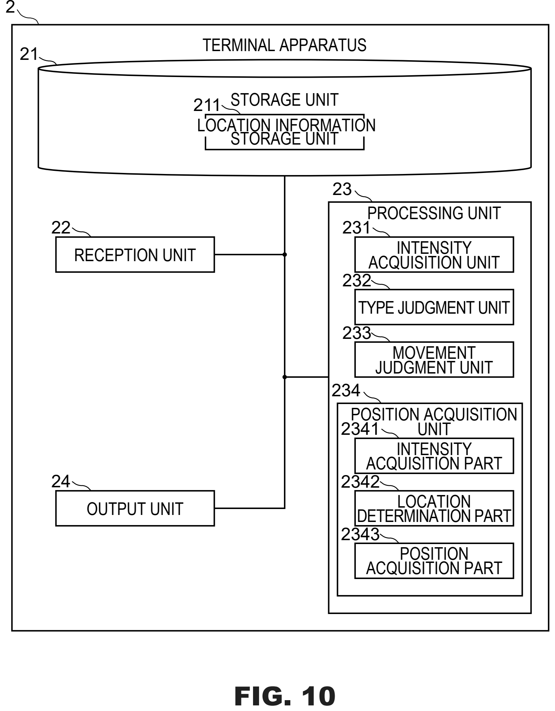

FIG. 10 is a block diagram for each terminal apparatus 2 according to the present embodiment. Each terminal apparatus 2 includes a storage unit 21, a reception unit 22, a processing unit 23, and an output unit 24. The storage unit 21 includes a location information storage unit 211. The processing unit 23 includes an intensity acquisition unit 231, a type judgment unit 232, a movement judgment unit 233, and a position acquisition unit 234. The position acquisition unit 234 includes an intensity acquisition part 2341, a location determination part 2342, and a position acquisition part 2343. The output unit 24 includes a position output unit 241.

The storage unit 21 stores various kinds of information. Examples of the various kinds of information include location information, which will be described later.

The location information storage unit 211 stores three or more pieces of location information. It is preferable that the three or more pieces of location information in the location information storage unit 211 are pieces of information accumulated by the location information production apparatus 1.

For example, each of the three or more pieces of location information in the location information storage unit 211 contains position information regarding a specific location, an apparatus identifier, and a radio wave intensity. It is preferable that each of the three or more pieces of position information is associated with three or more pieces of radio wave intensity information. Radio wave intensity information contains an apparatus identifier and a radio wave intensity. The three or more pieces of radio wave intensity information may constitute a radio wave intensity vector. The radio wave intensity vector is a vector using radio wave intensity information, and has a structure represented by (the radio wave intensity with the apparatus identifier 1, the radio wave intensity with the apparatus identifier 2, the radio wave intensity with the apparatus identifier 3, ..., the radio wave intensity with the apparatus identifier n). The location information storage unit 211 stores, for example, a location information management table having the structure shown in FIG. 8.

Each terminal apparatus 2 does not need to include the location information storage unit 211. In such a case, each terminal apparatus 2 references the location information storage unit 211 of an external apparatus (not shown) to acquire a terminal position, which will be described later.

The reception unit 22 receives, from each of three or more communication apparatuses B, a radio wave containing an apparatus identifier that identifies the communication apparatus B. The reception unit 22 typically has the same function as the above reception unit 13. The reception unit 22 typically receives radio waves containing apparatus identifiers from three or more communication apparatuses B. The reception unit 22 typically successively receive radio waves.

The processing unit 23 performs various kinds of processing. The various kinds of processing are, for example, the processing performed by the intensity acquisition unit 231, the type judgment unit 232, the movement judgment unit 233, and the position acquisition unit 234.

The intensity acquisition unit 231 acquires time-series radio wave intensities for each of the three or more communication apparatuses B. The intensity acquisition unit 231 acquires radio wave intensities based on the radio waves received by the reception unit 22. Note that the intensity acquisition unit 231 has the same function as the above intensity acquisition unit 141.

The type judgment unit 232 judges whether or not each of the three or more communication apparatuses B is a fixed terminal or a mobile terminal, using the time-series radio wave intensities acquired by the intensity acquisition unit 231. The type judgment unit 232 has the same function as the above type judgment unit 142.

The movement judgment unit 233 judges whether the terminal apparatus 2 is moving or stopped, and acquires a movement judgment result, which is the result of the judgment. The movement judgment result is, for example, "moving" or "stopped".

The movement judgment unit 233 acquires, for example, sensor information of the terminal apparatus 2, and use the sensor information to acquire the movement judgment result. The sensor information is, for example, acceleration measured by a gyro or time series position information.

For example, if the acceleration acquired by the gyro is "0" or not greater than a threshold value, the movement judgment unit 233 acquires the movement judgment result "stopped". If the acceleration acquired by the gyro is not less than a threshold value or greater than a threshold value, the movement judgment unit 233 acquires the movement judgment result "moving".

For example, using the time-series radio wave intensities of the three or more communication apparatuses B acquired by the intensity acquisition unit 231, the movement judgment unit 233 judges that the terminal apparatus is stopped if there is no change in the time-series radio wave intensities of one or more communication apparatuses B, and acquires a movement judgment result "stopped."

The position acquisition unit 234 acquires the radio wave intensities of three or more communication apparatuses B judged to be fixed terminals by the type judgment unit 232, and acquires terminal position, which is position information regarding the terminal apparatus 2 indoors, using the radio wave intensities of the fixed terminals.

For example, the position acquisition unit 234 acquires the radio wave intensities of three or more communication apparatuses B determined to be fixed terminals by the type judgment unit 232, references three or more pieces of location information in the location information storage unit 211, using the three or more radio wave intensities, and acquires the terminal position, using the fingerprinting method.

It is preferable that the position acquisition unit 234 uses the movement judgment result to acquire the position information. For example, it is preferable that the position acquisition unit 234 acquires the terminal position only when the movement judgment result indicates that the terminal apparatus is "stopped".

The position acquisition unit 234 may acquire the terminal position only when the apparatus identifier corresponding to the radio waves received by the reception unit 22 is contained in the location information in the location information storage unit 211, using the location information containing the apparatus identifier. This is because the location information in the location information storage unit 211 is location information regarding a fixed terminal.

The intensity acquisition part 2341 acquires the radio wave intensities of the three or more communication apparatuses judged to be fixed terminals by the type judgment unit 232, in association with the apparatus identifiers.

The location determination part 2342 determines, from among the pieces of location information in the location information storage unit 211, one or more pieces of location information that meet radio wave intensities associated with the three or more apparatus identifiers, acquired by the intensity acquisition part 2341, and a similarity condition.

For example, the location determination part 2342 acquires a first signal intensity vector, which is a vector having as elements the radio wave intensities paired with the three or more apparatus identifiers contained in the location information. In addition, for example, the location determination part 2342 acquires a second radio wave intensity vector, which is a vector having as elements the radio wave intensities associated with the three or more apparatus identifiers acquired by the intensity acquisition part 2341. For example, the location determination part 2342 acquires the degree of similarity between the two radio wave intensity vectors, and acquires the location information corresponding to the first radio wave intensity vector if the degree of similarity is not less than a threshold value or greater than a threshold value.

The position acquisition part 2343 acquires one or more pieces of position information respectively contained in the one or more pieces of location information determined by the location determination part 2342, and acquires the terminal position using one or more pieces of position information.

The output unit 24 outputs various kinds of information. Examples of the various kinds of information here include a terminal position and an indoor map.

Here, "output" is a concept that encompasses displaying on a display screen, projection using a projector, printing by a printer, the output of a sound, transmission to an external apparatus, accumulation on a recording medium, delivery of a processing result to another processing apparatus or another program, and the like.

The position output unit 241 outputs the terminal position acquired by the position acquisition unit 234. For example, the position output unit 241 displays, on an indoor map, a design clearly indicating the position specified by the terminal position.

It is preferable that the storage unit 21 and the location information storage unit 211 are realized using a non-volatile recording medium, but it can be realized using a volatile recording medium.

There is no limitation on the process in which information is stored in the storage unit 21 or the like. For example, information may be stored in the storage unit 21 or the like via a recording medium, or information transmitted via a communication line or the like may be stored in the storage unit 21 or the like, or information input via an input device may be stored in the storage unit 21 or the like.

The reception unit 22 is typically realized using a wireless or wired communication means.

The processing unit 23, the intensity acquisition unit 231, the type judgment unit 232, the movement judgment unit 233, the position acquisition unit 234, the intensity acquisition part 2341, the location determination part 2342, and the position acquisition part 2343 can typically be realized using a processor, a memory, or the like. The processing procedures performed by the processing unit 23 and so on are typically realized using software, and the software is recorded on a recording medium such as a ROM. However, such processing procedures may be realized using hardware (a dedicated circuit). Note that the processor may be a CPU, an MPU, a GPU, or the like, and there is no limitation on the type thereof.

The output unit 24 and the position output unit 241 can be realized using the driver software of the output device such as a display or a speaker, the driver software of the output device and the output device, or the like.

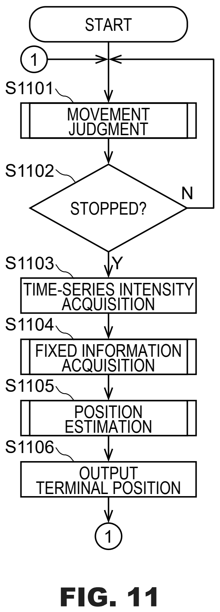

Next, a first operation example of a terminal apparatus 2 will be described with reference to the flowchart in FIG. 11.

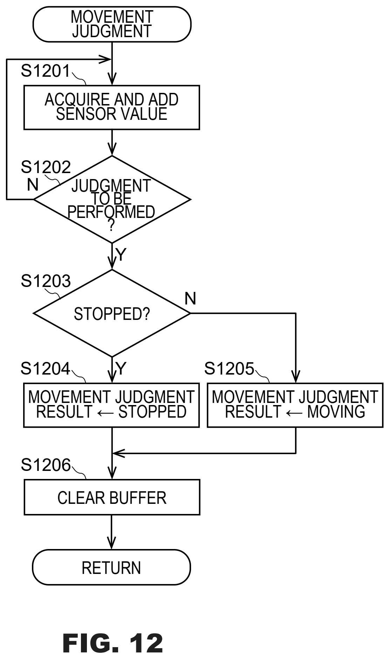

(Step S1101) The movement judgment unit 233 judges whether the terminal apparatus 2 is moving or stopped. An example of such movement judgment processing will be described with reference to the flowchart in FIG. 12.

(Step S1102) If the judgment result in step S1101 is "stopped", processing proceeds to step S1103, and if the judgment result is "moving", processing returns to step S1101.

(Step S1103) The intensity acquisition part 2341 performs time-series intensity acquisition processing. An example of time-series intensity acquisition processing has been described with reference to the flowchart in FIG. 4.

(Step S1104) The type judgment unit 232, the location determination part 2342, and so on perform fixed information acquisition processing. An example of fixed information acquisition processing has been described with reference to the flowchart in FIG. 5.

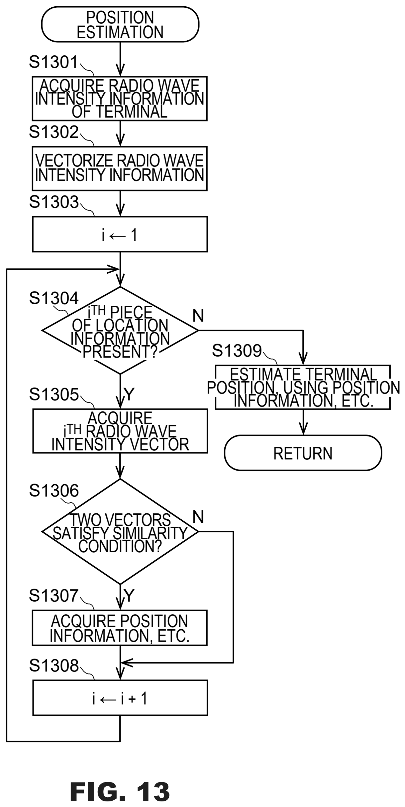

(Step S1105) The position acquisition part 2343 performs position estimation processing to acquire the terminal position. An example of position estimation processing will be described with reference to the flowchart in FIG. 13.

(Step S1106) The position output unit 241 outputs the terminal position acquired in step S1105. Processing returns to step S1101.

Note that, in the flowchart in FIG. 11, processing is terminated when power is turned off or an interruption is made to terminate the processing.

Next, an example of the movement judgment processing in step S1101 will be described with reference to the flowchart in FIG. 12.

(Step S1201) The movement judgment unit 233 acquires a sensor value (for example, acceleration) of the terminal apparatus 2 and temporarily accumulates it in a buffer (not shown).

(Step S1202) The movement judgment unit 233 judges whether or not to perform the movement judgment, using the sensor value in the buffer (not shown). If the movement judgment is to be performed, processing proceeds to step S1203, and if the movement judgment is not to be performed, processing returns to step S1201. The movement judgment unit 233 may always perform the movement judgment, or may perform the movement judgment after a predetermined number of sensor values more have been accumulated in the buffer, or after a predetermined time has elapsed since the acquisition of a sensor value, for example.

(Step S1203) The movement judgment unit 233 judges whether the terminal apparatus 2 is moving or stopped, using the one or more sensor values in the buffer (not shown). If the terminal apparatus 2 is stopped, the processing proceeds to step S1204, and if the terminal apparatus 2 is moving, processing proceeds to step S1205.

(Step S1204) The movement judgment unit 233 acquires the movement judgment result "stopped". Processing proceeds to step S1206.

(Step S1205) The movement judgment unit 233 acquires the movement judgment result "moving".

(Step S1206) The movement judgment unit 233 clears the buffer (not shown). Processing returns to higher level processing.

Next, an example of the position estimation processing in step S1105 will be described with reference to the flowchart in FIG. 13.

(Step S1301) The position acquisition part 2343 acquires three or more pieces of radio wave intensity information (sets of an apparatus identifier and a radio wave intensity) of the terminal apparatus 2.

(Step S1302) The position acquisition part 2343 vectorizes the three or more pieces of radio wave intensity information to acquire a radio wave intensity vector. The radio wave intensity vector is, for example, (the radio wave intensity with the apparatus identifier 1, the radio wave intensity with the apparatus identifier 2, the radio wave intensity with the apparatus identifier 3, ..., the radio wave intensity with the apparatus identifier n).

(Step S1303) The position acquisition part 2343 substitutes 1 for a counter i.

(Step S1304) The position acquisition part 2343 judges whether or not the i^{th} piece of location information is present in the location information storage unit 211. If the i^{th} piece of location information is present, processing proceeds to step S1305, and otherwise processing proceeds to step S1309.

(Step S1305) The position acquisition part 2343 acquires the i^{th} radio wave intensity vector contained in the i^{th} piece of location information from the location information storage unit 211.

(Step S1306) The position acquisition part 2343 acquires the degree of similarity between the radio wave intensity vector acquired in step S1302 and the i^{th} radio wave intensity vector acquired in step S1305. Next, the position acquisition part 2343 judges whether or not the degree of similarity satisfies the similarity condition (for example, the degree of similarity is not less than a threshold value). If the degree of similarity satisfies the similarity condition, processing proceeds to step S1307, and otherwise processing proceeds to step S1308.

(Step S1307) The position acquisition part 2343 acquires the position information contained in the i^{th} piece of location information and the degree of similarity, and accumulates them in a buffer (not sown).

(Step S1308) The position acquisition part 2343 increments the counter i by 1. Processing returns to step S1304.

(Step S1309) The position acquisition part 2343 acquires the terminal position that specifies the position of the terminal apparatus 2, using three or more sets, each consisting of a piece of position information and a degree of similarity accumulated in the buffer (not shown). Processing returns to higher level processing.

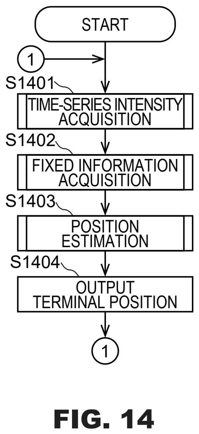

Next, a second operation example of a terminal apparatus 2 will be described with reference to the flowchart in FIG. 14.

(Step S1401) The intensity acquisition part 2341 performs time-series intensity acquisition processing. An example of time-series intensity acquisition processing has been described with reference to the flowchart in FIG. 4.

(Step S1402) The location determination part 2342 performs fixed information acquisition processing. An example of fixed information acquisition processing has been described with reference to the flowchart in FIG. 5.

(Step S1403) The position acquisition part 2343 performs position estimation processing to acquire the terminal position. An example of position estimation processing has been described with reference to the flowchart in FIG. 13.

(Step S1404) The position output unit 241 outputs the terminal position acquired in step S1403. Processing returns to step S1401.

Note that, in the flowchart in FIG. 14, processing is terminated when power is turned off or an interruption is made to terminate the processing.

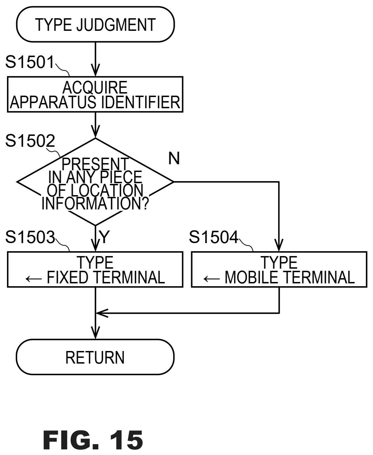

Next, a second example of type judgment processing in the fixed information acquisition processing in step S1402 of the flowchart in FIG. 14 will be described with reference to the flowchart in FIG. 15. The first example of type judgment processing has been described with reference to the flowchart in FIG. 6.

(Step S1501) The type judgment unit 232 acquires the apparatus identifier of the communication apparatus B to be subjected to the type judgment.

(Step S1502) The type judgment unit 232 judges whether or not the apparatus identifier acquired in step S1501 is present in any piece of location information in the location information storage unit 211. If the apparatus identifier is present in any piece of location information, processing proceeds to step S1503, and otherwise processing proceeds to step S1504.

(Step S1503) The type judgment unit 232 judges that the type is "fixed terminal". Processing returns to higher level processing.

(Step S1504) The type judgment unit 232 judges that the type is "mobile terminal". Processing returns to higher level processing.

Hereinafter, examples of specific operations of each terminal apparatus 2 according to the present embodiment will be described.

It is assumed that a user B, holding his/her terminal apparatus 2, enters an indoor location identified by a location identifier (P). It is assumed that the reception unit 22 of the terminal apparatus 2 transmits a location information request containing the location identifier (P) to an external apparatus (not shown), and receives the location information management table shown in FIG. 8 from the apparatus. It is assumed that the processing unit 23 thereafter temporarily accumulates the location information management table in the location information storage unit 211.

The terminal apparatus 2 operates as described below according to the processing from step S1103 to step S1106 in FIG. 11 or the processing in the flowchart in FIG. 14.

That is to say, the intensity acquisition part 2341 performs the time-series intensity acquisition processing described with reference to the flowchart in FIG. 4 to acquire the radio wave intensity of each of the three or more communication apparatuses B at the location X where the terminal apparatus 2 is present, and forms a time series radio wave intensity management table that has the structure shown in FIG. 7.

Next, the intensity acquisition part 2341 performs time series intensity acquisition processing, acquires time-series radio wave intensities of each of the communication apparatuses B that can receive radio waves at the location X, and forms the time-series radio wave intensity management table that has the structure shown in FIG. 7.

Next, with reference to the time-series radio wave intensity management table, the type judgment unit 142 judges whether each communication apparatus B is a "fixed terminal" or a "mobile terminal" according to the type judgment processing described with reference to the flowchart in FIG. 4.

Next, the location determination part 2342 acquires the radio wave intensities of "Apparatus 1", "Apparatus 3", "Apparatus 4", "Apparatus 6", and so on, which have been judged to be fixed terminals. Thereafter, the location determination part 2342 acquires a radio wave intensity vector "the radio wave intensity of the apparatus 1, the radio wave intensity of the apparatus 3, the radio wave intensity of the apparatus 4, the radio wave intensity of the apparatus 6, ...) = (P1,P3,P4,P6, ...). Here, note that the radio wave intensity of each communication apparatus B acquired by the location determination part 2342 may be a representative value of two or more radio wave intensity or one radio wave intensity such as the intensity of the latest radio wave of the communication apparatus B.

Next, the position acquisition part 2343 performs the position estimation processing described with reference to the flowchart in FIG. 13, and calculates the degree of similarity between the radio wave intensity vector at the location X and the radio wave intensity vector (vector constituted by pieces of radio wave intensity information) of each record in FIG. 8. Next, the position acquisition part 2343 determines the radio wave intensity vectors that satisfy the similarity condition "degree of similarity >= threshold value" (for example, the radio wave intensity vector (S₁₁,S₁₂,S₁₃, ...) with "ID = 1", the radio wave intensity vector (S₂₁,S₂₂,S₂₃, ...) with "ID = 2", and so on in FIG. 8). Next, the position acquisition part 2343 acquires sets of position information and a degree of similarity, paired with the radio wave intensity vector that satisfied the similarity condition (for example, "(x₁,y₁),DS₁", "(x₂,y₂),DS₂", and so on). Next, the position acquisition part 2343 acquires the indoor position (x₁ × DS₁ / the sum of the degrees of similarity + x₂ × DS₂ / the sum of the degrees of similarity + ..., y₁ × DS₁ / the sum of the degrees of similarity + y₂ × DS₂ / the sum of the degrees of similarity + ... ) at the location X. Note that the sum of the degrees of similarity is "DS₁ + DS₂ + ...".

Next, the position output unit 241 outputs the indoor or outdoor map stored in the storage unit 21, and places a pattern on the map at a position indicated by the position information (terminal position) acquired by the position acquisition part 2343.

As described above, according to the present embodiment, it is possible to easily acquire the positions of the terminal apparatuses 2 indoors or outdoors.

Note that the processing in the present embodiment may be realized using software. This software may be distributed through software downloading or the like. Also, this software may be recorded on a recording medium such as a CD-ROM and distributed. Note that the same applies to the other embodiments in the present description. Note that the software that realizes each terminal apparatus 2 according to the present embodiment is the program described below. That is to say, this program is a program that enables a computer to function as: a reception unit configured to receive, from each communication apparatus of three or more communication apparatuses, a radio wave containing an apparatus identifier identifying the communication apparatus; an intensity acquisition unit configured to acquire time-series radio wave intensities for each communication apparatus of the three or more communication apparatuses; a type judgment unit configured to judge, for each communication apparatus of the three or more communication apparatuses, whether the communication apparatus is a fixed terminal that is fixed or a mobile terminal that is moving, using the time-series radio wave intensities acquired by the intensity acquisition unit; a position acquisition unit configured to acquire a terminal position that is position information regarding the terminal apparatus, using radio wave intensities of three or more communication apparatuses judged to be fixed terminals by the type judgment unit; and a position output unit configured to output the terminal position acquired by the position acquisition unit.

### Third Embodiment

The present embodiment describes an action acquisition apparatus that acquires pieces of action information and time slots of a user, using position information of the user corresponding to a time, and outputs the pieces of action information associated with the time slots. It is preferable that the action acquisition apparatus further uses activity data and vital data of the user to acquire the action information and time slots of the user. The position information used when acquiring the action information may be the indoor position information acquired by the terminal apparatus 2 described in the second embodiment.

The present embodiment also describes an action acquisition apparatus that acquires action information, using past record information. It should be noted that the past record information may be information based on input from one or more users.

The present embodiment also describes an action acquisition apparatus that also acquires and outputs emotional information of a user. In the present embodiment, it is also preferable to use past record information to acquire emotion information.

In the present embodiment, the action acquisition apparatus is a terminal. However, as will be described in the fourth embodiment, the action acquisition apparatus may also be a server. In other words, the processing to acquire action information and emotion information, which will be described later, may be performed by the user's terminal or by the server.

The present embodiment also describes an action acquisition apparatus that uses a map to acquire and output place information when action information cannot be acquired.

Furthermore, the present embodiment describes an action acquisition apparatus that displays estimated action information and confirmed action information in a manner that allows them to be visually distinguished.

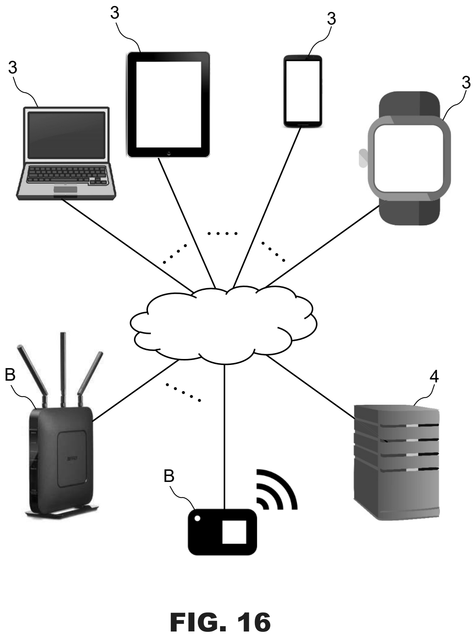

FIG. 16 is a conceptual diagram of an information system D according to the present embodiment. The information system D includes one or more action acquisition apparatuses 3, a server apparatus 4, and three or more communication apparatuses B.

Each action acquisition apparatus 3 is a terminal. Each action acquisition apparatus 3 is an apparatus that acquires and outputs action information. For example, each action acquisition apparatus 3 is a smartphone, a tablet terminal, a smartwatch, a so-called personal computer, or the like, and there is no limitation on the type thereof.

The server apparatus 4 is an apparatus that stores, for example, sets of action source information and action information of two or more users for each time slot. For example, the server apparatus 4 is an apparatus that stores learning information, which will be described later, and provides the learning information to the action acquisition apparatuses 3. For example, the server apparatus 4 is a cloud server or an ASP server, but there is no limitation of the type thereof.

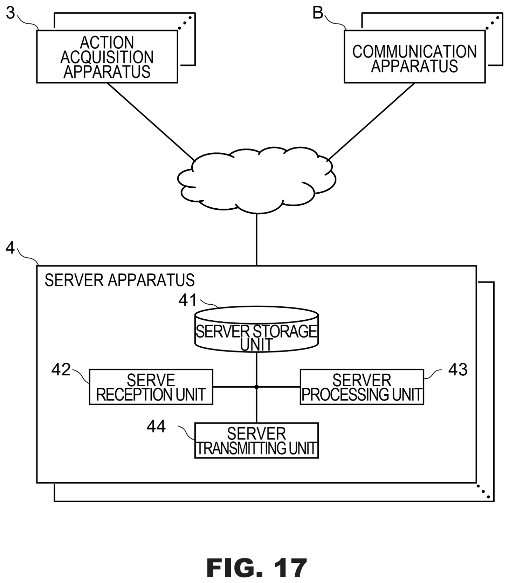

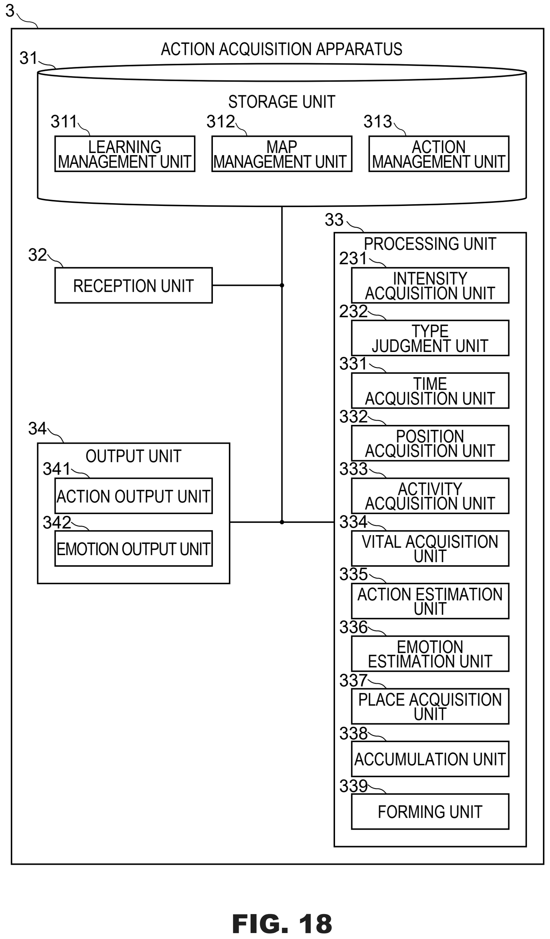

FIG. 17 is a block diagram of the information system D according to the present embodiment. FIG. 18 is a block diagram of each action acquisition apparatus 3.

The action acquisition apparatus 3 includes a storage unit 31, a reception unit 32, a processing unit 33, and an output unit 34. The storage unit 31 includes a learning management unit 311, a map management unit 312, and an action management unit 313. It should be noted that the action management unit 313 may be present in an external apparatus (not shown). The processing unit 33 includes the intensity acquisition unit 231, the type judgment unit 232, a time acquisition unit 331, a position acquisition unit 332, an activity acquisition unit 333, a vital acquisition unit 334, an action estimation unit 335, an emotion estimation unit 336, a place acquisition unit 337, an accumulation unit 338, and a forming unit 339. The output unit 34 includes an action output unit 341 and an emotion output unit 342.

The server apparatus 4 includes a server storage unit 41, a server reception unit 42, a server processing unit 43, and a server transmitting unit 44.

The action acquisition apparatus 3 receives, for example, an output instruction, a confirmation instruction, and input of information. The output instruction is an instruction to output output information, which will be described later. The output instruction typically includes period information. The period information is information that specifies the period in which the action information and so on are output. The confirmation instruction is an instruction to confirm the estimated action information or emotion information. The input of information is the input of information for changing the estimated action information or emotion information when the estimated action information or emotion information is incorrect. The input of information is input of updated action information or updated emotion information.

The storage unit 31 included in the action acquisition apparatus 3 stores various kinds of information. Examples of the various kinds of information include learning information, which will be described later, a map, which will be described later, action information, which will be described later, location information, calendar templates, action information corresponding to two or more action conditions, and emotion information corresponding to two or more emotion conditions.

A calendar template is information indicating the template of the calendar to be output. The calendar template may be, for example, an ICS file, a file written in HTML, or a file written in XML, but there is no limitation on the data structure thereof.

The action conditions are conditions for acquiring action information. The action conditions are conditions that each use two or more pieces of action source information. The action condition corresponds to the actional information. For example, an action condition is "position information = office AND 8:00 <= time <= 19:00", and the action information associated with this action condition is "work". For example, an action condition is "position information = kitchen AND activity data = standing AND 7:00 <= time <= 8:00", and the action information associated with this action condition is "cooking". For example, an action condition is "position information = park AND activity data = standing AND 120 <= heart rate", and the action information associated with this action condition is "running".

Emotion conditions are conditions for acquiring emotion information. Emotion conditions are each a condition that uses two or more pieces of emotion source information. Emotion conditions are associated with emotion information. For example, an emotion condition is "position information = office AND 8:00 <= time <= 19:00", and emotion information associated with this action condition is "positive". For example, an emotion condition is "position information = kitchen AND activity data = standing AND 7:00 <= time <= 8:00" and emotion information associated with this action condition is "positive". For example, an action condition is "position information = park AND activity data = standing AND 120 <= heart rate", and the emotion information associated with this action condition is "negative".

Learning information is stored in the learning management unit 311. Learning information is information based on two or more pieces of training data. The learning information in the learning management unit 311 is, for example, action learning information and emotion learning information. Each of the two or more pieces of learning information in the learning management unit 311 may be associated with a different user attribute value condition. The user attribute value conditions are conditions relating to one or more user attribute values.

The user attribute values are attribute values of the user. Examples of the user attribute values include occupation, family structure, marital status, sex, age, age group, whether the user is a morning person or a night person, and residential area, but there is no limitation.

The action learning information is information based on two or more pieces of action training data. The action learning information is, for example, an action learning model or an action correspondence table. The emotion learning information is information based on two or more pieces of emotion training data. The emotion learning information is, for example, an emotion learning model or an emotion correspondence table.

Action training data contains, for example, one or more pieces of action source information and action information. Emotion training data contains, for example, one or more pieces of action source information, action information, and emotion information.

Emotion training data contains, for example, one or more pieces of action source information or action information, and emotion information. Emotion training data contains, for example, one or more pieces of action source information, action information, and emotion information. In the emotion training data, one or more pieces of action source information, action information, or one or more pieces of action source information and action information are illustrative variables, and emotion information is an objective variable.

The action information is information that specifies the action of the user. Examples of the action information include "work", "watching TV", "walking", "running", "gym", "bath", and "sleep".

Emotion information is information relating to the user's emotions. The emotion information is, for example, positive (e.g., "1") or negative (e.g., "0"). The emotion information is, for example, the degree of positivity or the degree of negativity. The emotion information is, for example, joy (e.g., "1"), anger (e.g., "2"), sadness (e.g., "3"), or pleasure (e.g., "4").

The action source information is information that is the source for acquiring action information. The action source information includes position information. It is preferable that the action source information includes activity data or one or more kinds of vital data. The action source information may include emotion information. The action source information may include one or more pieces of past action information. Past action information typically includes immediately preceding action information. The action source information may include one or more pieces of future plan information of the user. Future plan information is, for example, information stored in a calendar server (not shown) (e.g., a Google Calendar (registered trademark) server). The action source information may include the time elapsed since the user arrived at the position indicated by the same position information. The action source information may include one or more user attribute values.

The position information is information that specifies the position of the action acquisition apparatus 3. The position information is, for example, (latitude,longitude), (latitude,longitude,altitude), a three-dimensional indoor relative position (x,y,z), a two-dimensional indoor relative position (x,y), or place information. Place information is information that expresses the meaning of a place. Place information is, for example, indoor place information or outdoor place information. Indoor place information is information that specifies an indoor place. Examples of indoor place information include "living room", "kitchen", "work room", and "office". Outdoor place information is information that specifies an outdoor place. Examples of outdoor place information include "ABC station", "library", "izakaya", and "location A".

Physical data is information regarding the user's body. Examples of physical data include activity data and vital data.

Activity data is information that specifies the user' activity. Examples of activity data include "standing" and "on the ground (e.g., sitting)".

Vital data is information that can be obtained from the user's biological state. Vital data can also be referred to as biometric information. Examples of vital data include heart rate and heart rate variability per unit time (e.g., 1 minute or 30 seconds), blood pressure (systolic and/or diastolic), respiration rate per unit time, and body temperature. The learning information is, for example, a learning model or a correspondence table. A learning model is information formed through machine learning processing using two or more pieces of training data, and is information used in machine learning prediction processing. The learning model may also be referred to as a learner, a classifier, a classification model, or the like. The machine learning algorithms can be any algorithm, such as deep learning, random forest, decision tree, SVM, or the like. In addition, for machine learning, for example, the TensorFlow library, the R language random forest module, various machine learning functions such as TinySVM, or various existing libraries can be used. In addition, when the learning information is a learning model, one or more pieces of action source information of the training data are illustrative variables, and the action information is an object variable.

The learning model here is, for example, an action learning model or an emotion learning model. The action learning model is a learning model for acquiring action information, and is information acquired through machine learning processing using action training data. The action training data contains one or more pieces of action source information and action information.

The emotion learning model is a learning model for acquiring emotion information, and is information acquired through machine learning processing using emotion training data. The emotion training data contains one or more pieces of emotion source information and emotion information.

The correspondence table is an action correspondence table or an emotion correspondence table. The action correspondence table is a table for acquiring action information. The action correspondence table contains two or more pieces of action correspondence information. The action correspondence information is information indicating the correspondence between one or more pieces of action source information and action information. The one or more pieces of action source information have, for example, a vector structure. Such a vector is referred to as an action source vector. The action source vector is a vector having one or more pieces of action source information as elements. The emotion correspondence table contains two or more pieces of emotion correspondence information. The emotion correspondence information is information indicating the correspondence between one or more pieces of emotion source information and emotion information. The one or more pieces of emotion source information have, for example, a vector structure. Such a vector is referred to as an emotion source vector. The emotion source vector is a vector having one or more pieces of emotion source information as elements.

The map management unit 312 stores a map. The map has place information corresponding to one or more pieces of position information. The map is, for example, in the KIWI format, but there is no limitation on the structure thereof.

The action management unit 313 stores action information associated with two or more time slots. The action information here is, for example, information acquired by the action estimation unit 335. The action information is information acquired by the action estimation unit 335 and changed by the user.

It is preferable that the action information here be can be distinguished as to whether or not it is confirmed action information. The action information is associated with, for example, a confirmation flag. The confirmation flag is a flag indicating that the action information has been confirmed. Whether or not the action information has been confirmed can be distinguished means that the storage areas for confirmed action information and for action information that has not been confirmed are different, for example. There is no limitation on the method for enabling discrimination as to whether or not the action information has been confirmed.

The reception unit 32 receives radio waves including an apparatus identifier that identifies a communication apparatus B from one or more communication apparatuses B. The reception unit 32 typically receives radio waves from three or more communication apparatuses B. The reception unit 32 has the same functions as the reception unit 22.

The processing unit 33 performs various kinds of processing. The various kinds of processing are, for example processing performed by the intensity acquisition unit 231, the type judgment unit 232, the time acquisition unit 331, and so on.

The time acquisition unit 331 acquires the time. The time acquisition unit 331 acquires the time from, for example, a clock (not shown). The time acquisition unit 331 receives the time from, for example, the server apparatus 4 or an apparatus not shown. The time may be in hours, minutes, and seconds, or may be in hours and minutes. The time may include one or more pieces of information from "year", "month", and "day". The time acquisition unit 331 may also acquire the day of the week. The day of the week may be regarded as information included in the acquired time.

The position acquisition unit 332 acquires position information. The position information is associated with time. The position acquisition unit 332 typically acquires position information in association with the time acquired by the time acquisition unit 331. The position acquisition unit 332 may perform the same processing as the position acquisition unit 234. In particular, for example, when the action acquisition apparatus 3 is indoors and cannot receive a GPS signal, it is preferable that the position acquisition unit 332 performs the same processing as the position acquisition unit 234. It is preferable that the position acquisition unit 332 includes a GPS receiver. For example, the position acquisition unit 332 acquires position information using the GPS receiver. Such position information is absolute position information. The position information acquired by the position acquisition unit 332 may be either outdoor or indoor position information.

It is preferable that the position acquisition unit 332 acquires indoor position information using the radio wave intensities of the communication apparatuses B judged by the type judgment unit 232 to be fixed terminals. The radio wave intensities used here are preferably the radio wave intensities of three or more communication apparatuses B, but may be the radio wave intensity of one or more communication apparatuses B.

The activity acquisition unit 333 acquires activity data of the user associated with time. The activity acquisition unit 333 typically acquires activity data in association with the time acquired by the time acquisition unit 331. The processing performed to acquire activity data is a well-known technique.

The vital acquisition unit 334 acquires one or more kinds of vital data of the user associated with time. The vital acquisition unit 334 typically acquires one or more kinds of vital data associated with the time acquired by the time acquisition unit 331. The processing performed to acquire vital data is a well-known technique.

The action estimation unit 335 uses two or more pieces of action source information including position information associated with time to acquire action information that identifies the user's action in a time slot specified by the time included in the two or more pieces of action source information. It is preferable that the action source information also includes activity data. It is also preferable that the action source information also includes one or more kinds of vital data.

For example, the action estimation unit 335 detects an action condition satisfied by two or more pieces of action source information including position information associated with time, and acquires, from the storage unit 31, action information paired with the action condition.

For example, if the user is sitting at a desk at home, which is indoors, at 13:15, the action estimation unit 335 acquires activity information "work". For example, if the user is sitting in the living room of the user's home, which is indoors, at 20:17, the action estimation unit 335 acquires action information "watching TV". For example, if position information "izakaya" is acquired at 20:17, the action estimation unit 335 acquires the action information "drinking party".

The action estimation unit 335 may use the action learning information in the learning management unit 311 and two or more pieces of action source information including position information associated with time, to acquire action information for a time slot. Hereinafter, the processing performed by the action estimation unit 335 when the learning information is a learning model and when the learning information is a correspondence table will be described.

The action estimation unit 335 may acquire one or more user attribute values, acquire action learning information paired with the user attribute value condition satisfied by the one or more user attribute values from the learning management unit 311, and use the action learning information to acquire action information.

### (1) When the action learning information is an action learning model

The action estimation unit 335 acquires the learning model from the learning management unit 311. Furthermore, the action estimation unit 335 acquires a time and one or more pieces of action source information associated with the time. Next, the action estimation unit 335 provides the time, the action source information, and the learning model to a module that performs machine learning prediction processing, and executes the module to acquire action information.

If the score output by the module is not greater than a threshold value or less than a threshold value, the action estimation unit 335 does not need to acquire action information.

### (2) When the action learning information is an action correspondence table

The action estimation unit 335 acquires a time and action source information associated with the time. Next, the action estimation unit 335 acquires an action source vector having the time and the action source information as elements. Next, the action estimation unit 335 calculates the degrees of similarity between the action source vector and the action source vectors contained in the two or more pieces of action correspondence information contained in the action correspondence table. Next, the action estimation unit 335 acquires, from the action correspondence table, a piece of action information paired with the action source vector with the highest degree of similarity. Note that even if the degree of similarity is the highest, if the degree of similarity is not greater than a threshold value or less than a threshold value, the action estimation unit 335 does not need to acquire action information.

The emotion estimation unit 336 acquires emotion information related to the emotion of the user in a time slot, using action information or action source information.

For example, the emotion estimation unit 336 detects an emotion condition satisfied by two or more pieces of emotion source information including position information associated with the time, and acquires emotion information paired with the emotion condition from the storage unit 31.

For example, if the user is sitting at a desk, which is indoors, at home at 13:15, the emotion estimation unit 336 acquires emotion information "positive". For example, if the user is sitting in the living room of the user's home, which is indoors, at 20:17, the emotion estimation unit 336 acquires emotion information "positive". For example, if position information "izakaya" is acquired at 20:17, the emotion estimation unit 336 acquires emotion information "positive".

The emotion estimation unit 336 acquires emotion information for a time slot, using the emotion learning information in the learning management unit 311 and the action information acquired by the action estimation unit 335 or one or more pieces of action source information that are the basis for acquiring the action information. Here, the action information or one or more pieces of action source information are referred to as emotion source information because they are used to acquire emotion information.

The emotion estimation unit 336 may acquire one or more user attribute values, acquire emotion learning information paired with a user attribute value condition satisfied by the one or more user attribute values from the learning management unit 311, and acquire emotion information using the emotion learning information.

With respect to the emotion estimation unit 336, the following describes the processing performed by the action estimation unit 335 when the emotion learning information is an emotion learning model and when the emotion learning information is the emotion correspondence table.

### (1) When the emotion learning information is an emotion learning model

The emotion estimation unit 336 acquires the emotion learning model from the learning management unit 311. The emotion estimation unit 336 also acquires one or more pieces of action source information or action information associated with the time. Next, the emotion estimation unit 336 provides the acquired one or more pieces of action source information or action information and the emotion learning model to a module that performs machine learning prediction processing, and executes the module to acquire emotion information.

It should be noted that if the score output by the module is not greater than a threshold value or less than a threshold value, the emotion estimation unit 336 does not need to acquire emotion information.

### (2) When the emotion learning information is an emotion correspondence table

The emotion estimation unit 336 acquires one or more pieces of action source information or action information associated with the time. Next, the emotion estimation unit 336 acquires an emotion source vector having the one or more pieces of action source information or action information as elements. Next, the emotion estimation unit 336 calculates the degrees of similarity between the emotion source vector and the emotion source vectors contained in the two or more pieces of correspondence information contained in the emotion correspondence table. Next, the emotion estimation unit 336 acquires emotion information paired with the emotion source vector with the highest degree of similarity from the emotion correspondence table. Note that even if the degree of similarity is the highest, if the degree of similarity is not greater than the threshold value or less than the threshold value, the emotion estimation unit 336 does not need to acquire emotion information.

The place acquisition unit 337 references the map in the map management unit 312 and acquires place information corresponding to the position information acquired by the position acquisition unit 332. The position information in such a case is typically absolute position information (e.g., (latitude,longitude)).

It is preferable that the place acquisition unit 337 acquires place information only for a time slot for which the action estimation unit 335 did not acquire action information.

The accumulation unit 338 accumulates the action information acquired by the action estimation unit 335 in the action management unit 313 in association with the time slot.

The accumulation unit 338 may accumulate the emotion information acquired by the emotion estimation unit 336 in the action management unit 313 in association with the time slot.

The forming unit 339 forms information to be output, using the action information from the action management unit 313. For example, the forming unit 339 uses the emotion information from the action management unit 313 to form the information to be output.

For example, in areas specified by two or more time slots on the calendar, the forming unit 339 forms output information containing pieces of action information respectively paired with the time slots. It is preferable that two or more pieces of action information are arranged in the output information so that the confirmed action information and the unconfirmed action information can be visually distinguished from each other. It is preferable that the forming unit 339 forms output information that visually indicates emotion information corresponding to two or more time slots. It is preferable that the forming unit 339 forms output information in which, for example, time slots in which emotion information is "positive" and time slots in which emotion information is "negative" are visually distinguishably expressed. It is preferable that the forming unit 339 forms output information such that, for example, the background colors of time slots in which emotion information is "positive" and time slots in which emotion information is "negative" are different.

The output unit 34 outputs various kinds of information. Examples of the various kinds of information include action information, emotion information, and place information.

Here, "output" is typically displaying on a display screen, but may be a concept that encompasses projection using a projector, printing by a printer, the output of a sound, transmission to an external apparatus, accumulation on a recording medium, delivery of a processing result to another processing device or another program, and so on.

The action output unit 341 outputs action information for each of one or more time slots.

It is preferable that the action output unit 341 outputs the place acquired by the place acquisition unit 337 when the action estimation unit 335 cannot acquire action information.

It is preferable that the action output unit 341 outputs two or more pieces of action information so that the confirmed action information and the unconfirmed action information can be visually distinguished.

The emotion output unit 342 outputs emotion information. For example, the emotion output unit 342 outputs the emotion information acquired by the emotion estimation unit 336. It is preferable that the emotion output unit 342 outputs emotion information for each of one or more time slots.

The server storage unit 41 included in the server apparatus 4 stores various kinds of information. Examples of the various kinds of information include the above-mentioned learning information, pieces of action source information and times corresponding to two or more user identifiers, two or more pieces of action training data, and two or more pieces of emotion training data.

The server reception unit 42 receives various kinds of instructions and information. Examples of the various kinds of instructions and information include an instruction to transmit information. The information here is, for example, learning information and action source information.

The server processing unit 43 performs various kinds of processing. Examples of the various kinds of processing include learning processing. The learning processing is, for example, action learning processing or emotion learning processing.

Action learning processing is processing performed to acquire an action learning model using two or more pieces of action training data. For example, the server processing unit 43 provides two or more pieces of action training data to a machine learning processing module, and executes the module to acquire an action learning model, and accumulates it in the server storage unit 41. For example, the server processing unit 43 provides, for each of two or more action information candidates, two or more positive examples that are training data including the action information and two or more negative examples that are training data not including the action information to a machine learning processing module, executes the module, acquires an action learning model for each action information candidate, and accumulates it in the server storage unit 41 in association with the action information. For example, the server processing unit 43 acquires an action correspondence table, which is a table containing two or more pieces of action training data as records, and accumulates it in the server storage unit 41.

Emotion learning processing is processing performed to acquire an emotion learning model using two or more pieces of emotion training data. For example, the server processing unit 43 provides two or more pieces of emotion training data to a machine learning processing module, executes the module to acquire an emotion learning model, and accumulates it in the server storage unit 41. For example, the server processing unit 43 provides, for each of two or more emotion information candidates, two or more positive examples that are training data including the emotion information and two or more negative examples that are training data not including the emotion information to a machine learning processing module, executes the module, acquires an emotion learning model for each emotion information candidate, and accumulates it in the server storage unit 41 in association with the emotion information. For example, the server processing unit 43 acquires an emotion correspondence table, which is a table having two or more pieces of emotion training data as records, and accumulates it in the server storage unit 41.

The server transmitting unit 44 transmits various kinds of information. Examples of the various kinds of information include an action learning model, an emotion learning model, an action correspondence table, and an emotion correspondence table.

The storage unit 31, the learning management unit 311, the map management unit 312, the action management unit 313, and the server storage unit 41 are preferably non-volatile recording media, but they can also be realized using volatile recording media.

There is no limitation on the process in which information is stored in the storage unit 31 or the like. For example, information may be stored in the storage unit 31 or the like via a recording medium, or information transmitted via a communication line or the like may be stored in the storage unit 31 or the like, or information input via an input device may be stored in the storage unit 31 or the like.

The reception unit 32, the server reception unit 42, and the server transmitting unit 44 are typically realized using wireless or wired communication means.

The processing unit 33, the time acquisition unit 331 , the position acquisition unit 332, the activity acquisition unit 333, the vital acquisition unit 334, the action estimation unit 335, the emotion estimation unit 336, the place acquisition unit 337, the accumulation unit 338, the forming unit 339, and the server processing unit 43 can typically be realized using a processor, a memory, and so on. The processing procedures performed by the processing unit 33 and so on are typically realized using software, and the software is recorded on a recording medium such as a ROM. However, such processing procedures may be realized using hardware (a dedicated circuit). It should be noted that the processor may be a CPU, an MPU, a GPU, or the like, and there is no limitation on the type thereof.

The output unit 34, the action output unit 341, and the emotion output unit 342 may or may not be considered to include an output device such as a display or a speaker. The output unit 34 can be realized using the driver software of the output device, the driver software of the output device and the output device, or the like.

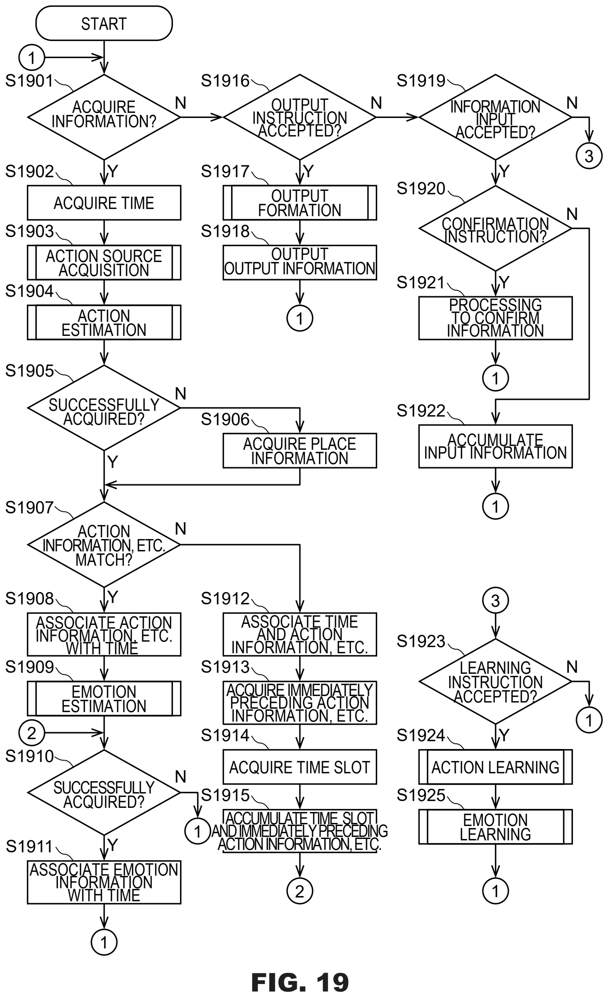

Next, an example of the operation of the action acquisition apparatus 3 included in the information system D will be described with reference to the flowchart in FIG. 19.

(Step S1901) The processing unit 33 judges whether or not to acquire information. If the information is to be acquired, processing proceeds to step S1902, and if the information is not to be acquired, processing proceeds to step S1916. The processing unit 33 may always judge to acquire information, or may judge to acquire information when, for example, a flag indicating that information is to be acquired is stored in the storage unit 31. There is no limitation on the conditions for such a judgment.

(Step S1902) The time acquisition unit 331 acquires the time from a clock (not shown). Here, the time acquisition unit 331 may also acquire the day of the week.

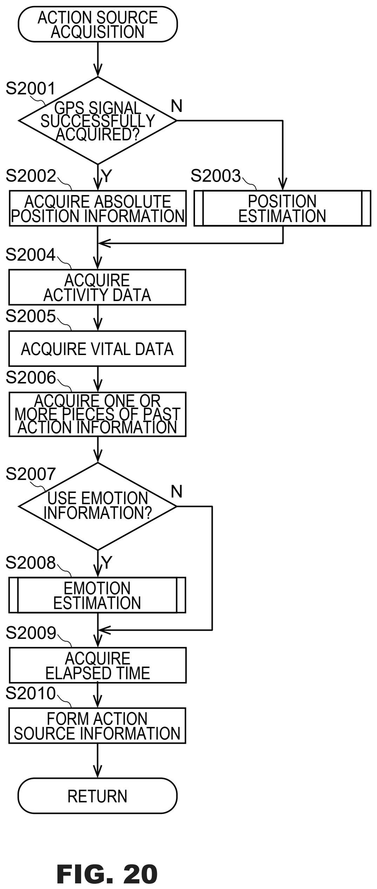

(Step S1903) The processing unit 33 acquires one or more pieces of action source information. An example of such action source acquisition processing will be described with reference to the flowchart in FIG. 20.

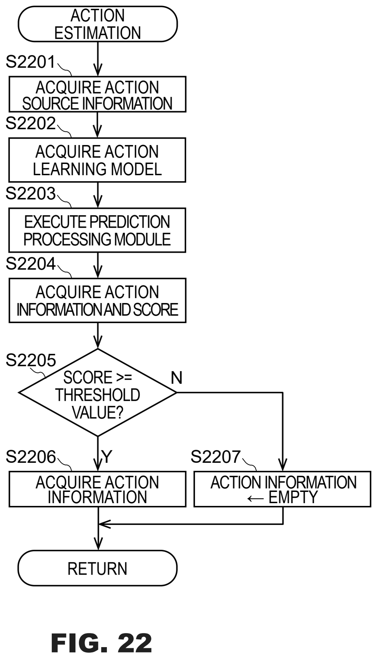

(Step S1904) The action estimation unit 335 estimates action information that specifies the user's action, using one or more pieces of action source information acquired in step S1903. An example of such action estimation processing will be described with reference to the flowcharts in FIGS. 22 to 24.

(Step S1905) The place acquisition unit 337 judges whether or not the action information has been successfully acquired in step S1904. If the action information has been successfully acquired, processing proceeds to step S1907, and if the action information has not been successfully acquired, processing proceeds to step S1906.

(Step S1906) The place acquisition unit 337 references the map in the map management unit 312 and acquires place information corresponding to the position information acquired in step S1903. It should be noted here that there may be cases where the place information cannot be acquired.

(Step S1907) The accumulation unit 338 judges whether or not the action information stored in a buffer (not shown), which is the action information most recently accumulated, matches the action information acquired in step S1904. If they match, processing proceeds to step S1908, and otherwise processing proceeds to step S1912.

(Step S1908) The accumulation unit 338 associates the acquired action information and the like with the time acquired in step S1902, and accumulates them in a buffer (not shown).

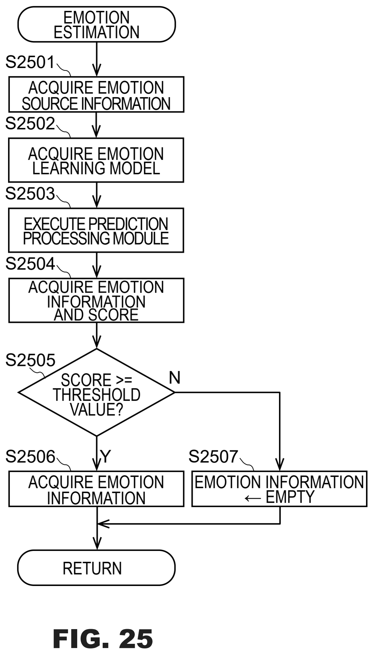

(Step S1909) The emotion estimation unit 336 performs processing to estimate emotion information. An example of such emotion estimation processing will be described with reference to the flowcharts in FIGS. 25 to 27.

(Step S1910) The accumulation unit 338 judges whether or not emotion information has been successfully acquired in step S1909. If emotion information has been successfully acquired, processing proceeds to step S1911, and if emotion information has not been successfully acquired, processing returns to step S1901.

(Step S1911) The accumulation unit 338 accumulates the emotion information acquired in step S1909 in a buffer (not shown) in association with the time acquired in step S1902. Processing returns to step S1901.

(Step S1912) The accumulation unit 338 accumulates the time acquired in step S1902 and the acquired action information and so on in a buffer (not shown) in association with each other.

(Step S1913) The accumulation unit 338 acquires the immediately preceding action information or the like.

(Step S1914) The accumulation unit 338 acquires time slots specified by two or more times associated with the immediately preceding action information or the like.

(Step S1915) The accumulation unit 338 accumulates the time slots acquired in step S1914 and the immediately preceding action information acquired in step S1913 in the action management unit 313 in association with each other. It should be noted here that the accumulation unit 338 may accumulate the time slots and the immediately preceding action information or the like in association with the user identifier. In such a case, it is preferable that the accumulation destination is the server apparatus 4.

(Step S1916) The action acquisition apparatus 3 judges whether or not an output instruction has been accepted. If an output instruction has been accepted, processing proceeds to step S1917, and otherwise processing proceeds to step S1919.

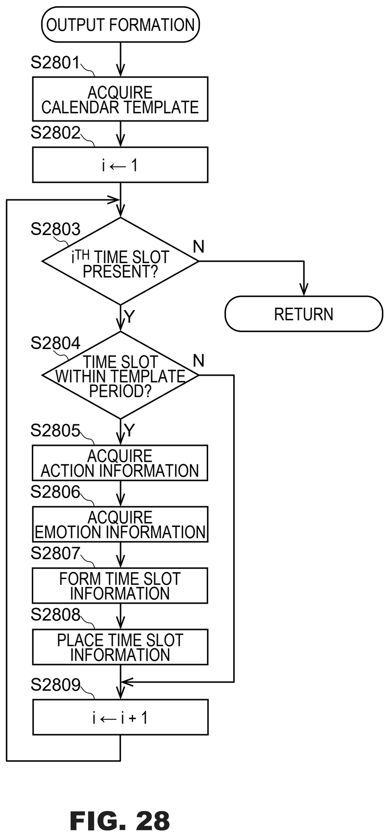

(Step S1917) The forming unit 339 forms output information using the action information in the action management unit 313 and the like. An example of such output forming processing will be described with reference to the flowchart in FIG. 28.

(Step S1918) The output unit 34 outputs the output information formed in step S1917. Processing returns to step S1901.

(Step S1919) The action acquisition apparatus 3 judges whether or not input of information has been accepted in response to the output information being output. If the input of information has been accepted, processing proceeds to step S1920, and otherwise processing proceeds to step S1923.

(Step S1920) The processing unit 33 judges whether or not the information accepted in step S1919 is a confirmation instruction made to confirm estimated action information or estimated emotion information. If the information is a confirmation instruction, processing proceeds to step S1921, and if the information is not a confirmation instruction, processing proceeds to step S1922.

(Step S1921) The accumulation unit 338 performs processing to confirm the action information or emotion information in response to the confirmation instruction. Processing returns to step S1901. It should be noted that such processing is, for example, processing performed to associate a confirmation flag with action information corresponding to the confirmation instruction or emotion information corresponding to the confirmation instruction.

(Step S1922) The accumulation unit 338 accumulates the input information. Processing returns to step S1901. It should be noted that the input information is, for example, correct action information or correct emotion information. Thereafter, the accumulation unit 338 updates the estimated action information or emotion information corresponding to the input information to the input action information or emotion information. In addition, the accumulation unit 338 performs processing to confirm such action information or emotion information.

(Step S1923) The action acquisition apparatus 3 judges whether or not a learning instruction has been accepted. If a learning instruction has been accepted, processing proceeds to step S1924, and otherwise processing returns to step S1901.

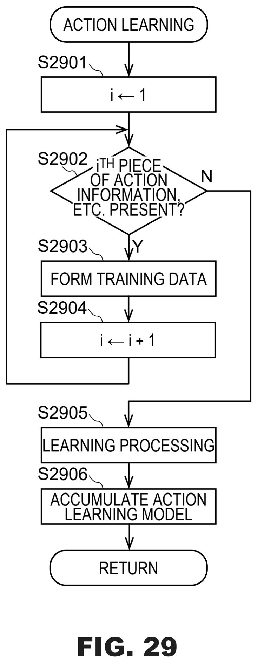

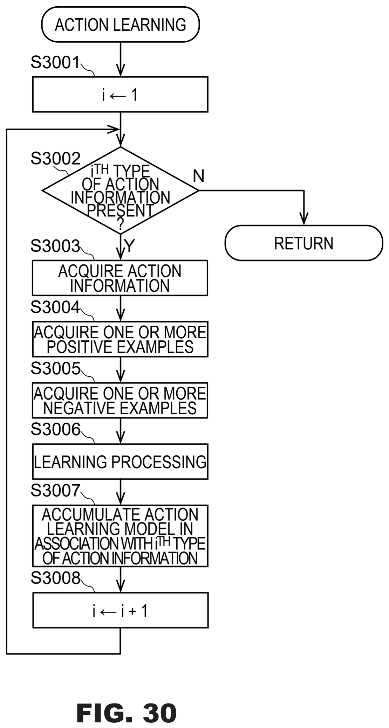

(Step S1924) A learning unit (not shown) or a learning apparatus (not shown) of the action acquisition apparatus 3 forms action learning information, using two or more pieces of action training data including action information or the like, and accumulates it in the learning management unit 311. An example of such action learning processing will be described with reference to the flowcharts in FIGS. 29 and 30.

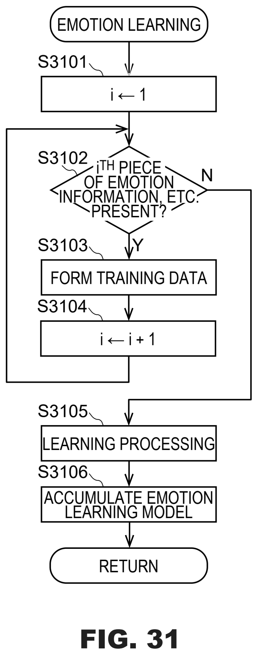

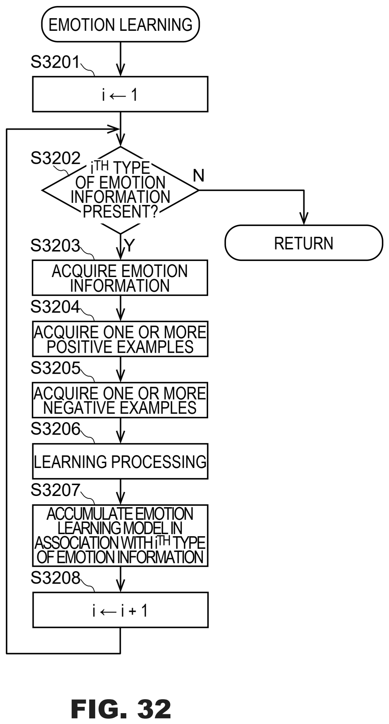

(Step S1925) A learning unit (not shown) or a learning apparatus (not shown) of the action acquisition apparatus 3 forms emotion learning information, using two or more pieces of emotion training data including emotion information or the like, and accumulates it in the learning management unit 311. Processing returns to step S1901. An example of such emotion learning processing will be described with reference to the flowcharts in FIGS. 31 and 32.

It should be noted that in the flowchart in FIG. 19, processing is terminated when power is turned off or an interruption is made to terminate the processing.

Next, an example of the action source acquisition processing in step S1903 will be described with reference to the flowchart in FIG. 20.

(Step S2001) The processing unit 33 judges whether or not the reception unit 32 has successfully acquired a GPS signal. If a GPS signal has been successfully acquired, processing proceeds to step S2002, and if a GPS signal has not been successfully acquired, processing proceeds to step S2003.

(Step S2002) The position acquisition unit 332 acquires absolute position information based on the GPS signal received by the reception unit 32.

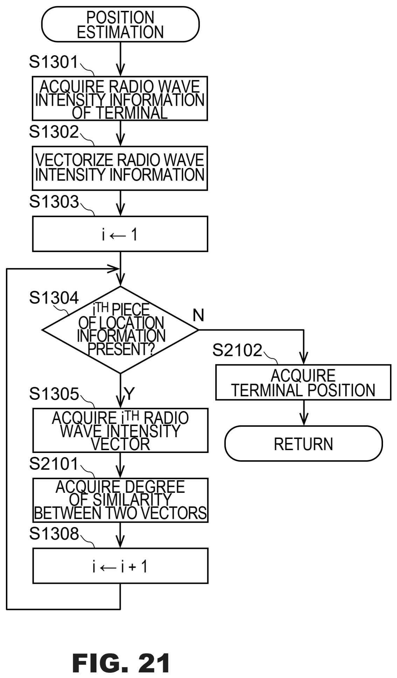

(Step S2003) The position acquisition unit 332 acquires position information. An example of such position estimation processing will be described with reference to the flowchart in FIG. 21.

(Step S2004) The activity acquisition unit 333 acquires the activity data of the user.

(Step S2005) The vital acquisition unit 334 acquires one or more kinds of vital data of the user.

(Step S2006) The action estimation unit 335 acquires one or more pieces of past action information. It should be noted that the one or more pieces of past action information include action information that is immediately preceding in time.

(Step S2007) The action estimation unit 335 judges whether or not to use emotion information for action estimation processing. If emotion information is to be used, processing proceeds to step S2008, and if emotion information is not to be used, processing proceeds to step S2009. It should be noted that whether or not to use emotion information for action estimation processing is typically determined in advance.

(Step S2008) The emotion estimation unit 336 acquires emotion information. An example of such emotion estimation processing will be described with reference to the flowcharts in FIGS. 25 to 27.

(Step S2009) The action estimation unit 335 acquires the elapsed time since a new action, specified by a new pieces of action information, has started.

(Step S2010) The action estimation unit 335 forms information to be used in action estimation processing, using two or more kinds of action source information including time and position information. Processing returns to the higher-level processing. Here, the information to be formed is typically a set of pieces of action source information, and is, for example, an action source vector having two or more pieces of action source information as elements. The two or more kinds of action source information are, for example, two or more kinds of information selected from the group consisting of time, day of the week, position information, activity data, vital data, past action information, emotion information, and elapsed time.

It should be noted here that in the flowchart in FIG. 20, even if a GPS signal has been successfully acquired, the position acquisition unit 332 may acquire position information through position estimation processing, which will be described with reference to the flowchart in FIG. 21.

In the flowchart in FIG. 20, the action estimation unit 335 may acquire one or more user attribute values, and acquire action source information including the one or more user attribute values. It should be noted that the user attribute values may be information stored in the storage unit 31 or information or the like input by the user. Next, an example of the position estimation processing in step S2003 will be described with reference to the flowchart in FIG. 21. In the flowchart in FIG. 21, the description of the same steps as those in FIG. 13 will be omitted. In addition, the position estimation processing in step S2003 may be the same as the processing in the flowchart in FIG. 13.

(Step S2101) The position acquisition unit 332 acquires the degree of similarity between two radio wave intensity vectors and temporarily accumulates the degree of similarity in a buffer (not shown) in association with the i^{th} piece of location information. Processing proceeds to step S1308.

(Step S2102) The position acquisition unit 332 acquires the position information contained in the location information paired with the highest degree of similarity. Processing returns to the higher-level processing. It should be noted that the acquired position information is the terminal position.

Next, an example of the first action estimation processing in step S1904 will be described with reference to the flowchart in FIG. 22. The flowchart in FIG. 22 shows processing performed to estimate action information through machine learning prediction processing using one action learning model. In other words, the flowchart in FIG. 22 shows processing performed to estimate action information through multi-value classification prediction processing in machine learning.

(Step S2201) The action estimation unit 335 acquires the two or more kinds of action source information (e.g., action source vectors) acquired in step S1903.

(Step S2202) The action estimation unit 335 acquires an action learning model from the learning management unit 311.

(Step S2203) The action estimation unit 335 provides the two or more kinds of action source information and the action learning model acquired in step S2201 to a machine learning prediction processing module, and executes the module.

(Step S2204) The action estimation unit 335 acquires estimated action information and a score, which are the results of the execution in step S2203.

(Step S2205) The action estimation unit 335 judges whether or not the score acquired in step S2204 is not less than a threshold value. If the score is not less than the threshold value, processing proceeds to step S2206, and if the score is less than the threshold value, processing proceeds to step S2207.

(Step S2206) The action estimation unit 335 acquires the action information acquired in step S2204 as action information to be output. Processing returns to the higher-level processing.

(Step S2207) The action estimation unit 335 acquires "empty" action information. Processing returns to the higher-level processing.

It should be noted that in the flowchart in FIG. 22, the processing from step S2205 to step S2207 do not necessarily have to be performed.

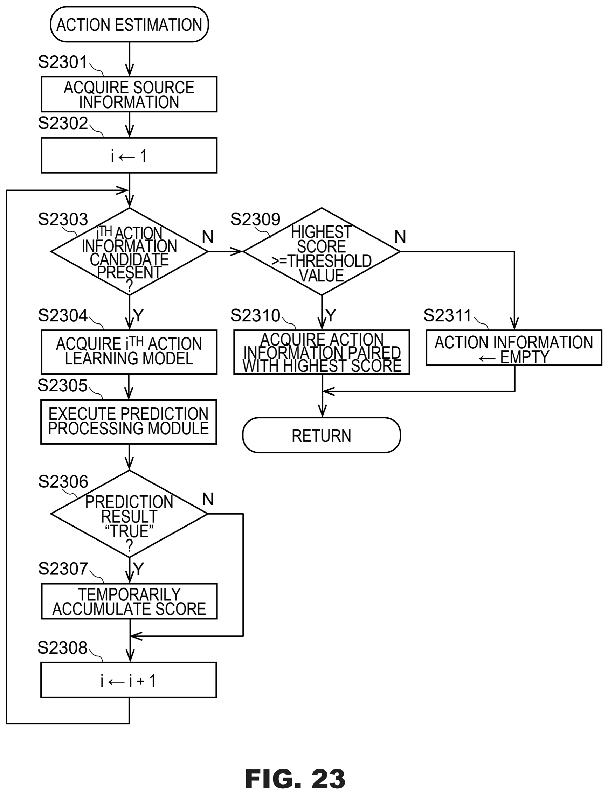

Next, an example of the second action estimation processing in step S1904 will be described with reference to the flowchart in FIG. 23. The flowchart in FIG. 23 shows processing performed to estimate action information through machine learning prediction processing using an action learning model for each of two or more action information candidates. In other words, the flowchart in FIG. 22 shows processing performed to estimate action information through binary classification prediction processing in machine learning.

(Step S2301) The action estimation unit 335 acquires the two or more kinds of action source information acquired in step S1903.

(Step S2302) The action estimation unit 335 assigns 1 to a counter i.

(Step S2303) The action estimation unit 335 judges whether or not the i^{th} action information candidate is present with reference to the learning management unit 311. If the i^{th} action information candidate is present, processing proceeds to step S2304, and otherwise processing proceeds to step S2309.

(Step S2304) The action estimation unit 335 acquires the i^{th} action learning model paired with the i^{th} action information candidate from the learning management unit 311.

(Step S2305) The action estimation unit 335 provides the two or more kinds of action source information acquired in step S2301 and the i^{th} action learning model acquired in step S2304 to a prediction processing module that performs binary classification in machine learning, and executes the module.

(Step S2306) The action estimation unit 335 judges whether or not the execution result in step S2305 is "true" or not. If the execution result is "true", processing proceeds to step S2307, and if the execution result is "false", processing proceeds to step S2308.

(Step S2307) The action estimation unit 335 temporarily accumulates the score, which is part of the execution results in step S2305, in a buffer (not shown) in association with the i^{th} action information candidate.

(Step S2308) The action estimation unit 335 increments the counter i by one. Processing returns to step S2303.

(Step S2309) The action estimation unit 335 acquires the highest score and judges whether or not the score is not less than a threshold value. If the highest score is not less than the threshold value, processing proceeds to step S2310, and if the highest score is less than the threshold value, processing proceeds to step S2311.

(Step S2310) The action estimation unit 335 acquires the action information paired with the highest score. Processing returns to the higher-level processing.

(Step S2311) The action estimation unit 335 acquires "empty" action information. Processing returns to the higher-level processing.

In the flowchart in FIG. 23, the processing from step S2308 to step S2311 do not necessarily have to be performed.

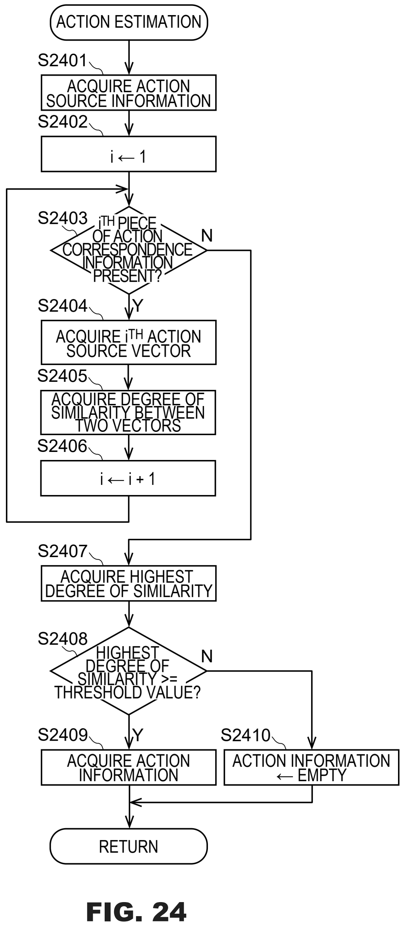

Next, an example of the third action estimation processing in step S1904 will be described with reference to the flowchart in FIG. 24. The flowchart in FIG. 24 shows processing performed to estimate action information using an action correspondence table.

(Step S2401) The action estimation unit 335 acquires the two or more kinds of action source information acquired in step S1903. The two or more kinds of action source information here are action source vectors.

(Step S2402) The action estimation unit 335 assigns 1 to a counter i.

(Step S2403) The action estimation unit 335 judges whether or not the i^{th} piece of action correspondence information is present in the action correspondence table in the learning management unit 311. If the i^{th} piece of action correspondence information is present, processing proceeds to step S2404, and otherwise processing proceeds to step S2407.

(Step S2404) The action estimation unit 335 acquires the i^{th} piece of action source vector contained in the i^{th} piece of action correspondence information.

(Step S2405) The action estimation unit 335 acquires the degree of similarity between the action source vector acquired in step S2403 and the action source vector acquired in step S2404, and associates the degree of similarity with the i^{th} piece of action correspondence information.

(Step S2406) The action estimation unit 335 increments the counter i by one. Processing returns to step S2403.

(Step S2407) The action estimation unit 335 acquires the highest degree of similarity.

(Step S2408) The action estimation unit 335 judges whether or not the highest degree of similarity acquired in step S2407 is not less than a threshold value. If the highest degree of similarity is not less than the threshold value, processing proceeds to step S2409, and if the highest degree of similarity is less than the threshold value, processing proceeds to step S2410.

(Step S2409) The action estimation unit 335 acquires a piece of action information associated with the i^{th} piece of action correspondence information paired with the highest degree of degree of similarity. Processing returns to the higher-level processing.

(Step S2410) The action estimation unit 335 acquires "empty" action information. Processing returns to the higher-level processing.

It should be noted that in the flowchart in FIG. 24, the processing from step S2408 to step S2411 do not necessarily have to be performed.

Next, an example of the first emotion estimation processing in step S1909 will be described with reference to the flowchart in FIG. 25. The flowchart in FIG. 25 shows processing performed to estimate emotion information through machine learning prediction processing using one emotion learning model. In other words, the flowchart in FIG. 25 shows processing performed to estimate emotion information through multi-value classification prediction processing in machine learning.

(Step S2501) The emotion estimation unit 336 acquires the two or more kinds of action source information acquired in step S1903. The two or more kinds of action source information here are pieces of emotion source information. The two or more kinds of emotion source information are, for example, emotion source vectors.

(Step S2502) The emotion estimation unit 336 acquires an emotion learning model from the learning management unit 311.

(Step S2503) The emotion estimation unit 336 provides the two or more kinds of emotion source information acquired in step S2501 and the emotion learning model to a machine learning prediction processing module, and executes the module.

(Step S2504) The emotion estimation unit 336 acquires estimated emotion information and a score, which are the execution results in step S2503.

(Step S2505) The emotion estimation unit 336 judges whether or not the score acquired in step S2504 is not less than a threshold value. If the score is not less than the threshold value, processing proceeds to step S2506, and if the score is less than the threshold value, processing proceeds to step S2507.

(Step S2506) The emotion estimation unit 336 acquires the emotion information acquired in step S2504 as emotion information to be output. Processing returns to the higher-level processing.

(Step S2507) The emotion estimation unit 336 acquires "empty" emotion information. Processing returns to the higher-level processing.

It should be noted that the in the flowchart in FIG. 25, the processing from step S2505 to step S2507 do not necessarily have to be performed.

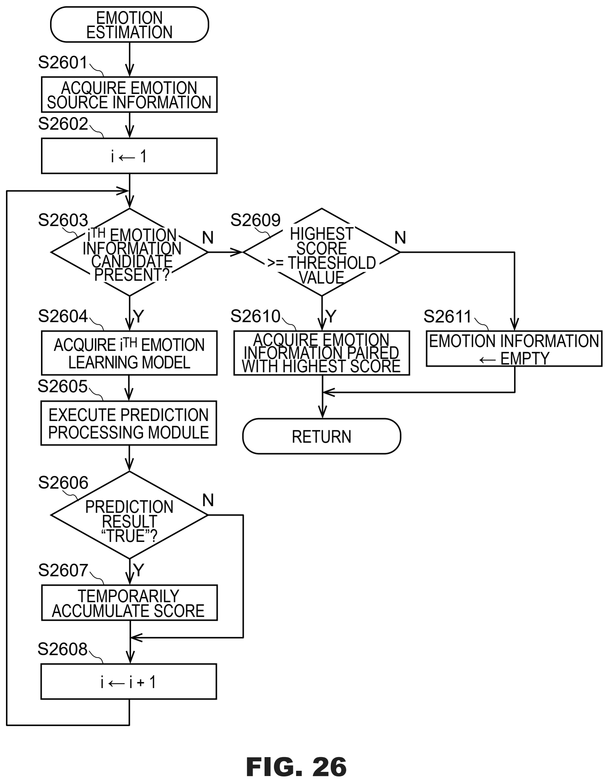

Next, an example of the second emotion estimation processing in step S1909 will be described with reference to the flowchart in FIG. 26. The flowchart in FIG. 26 shows processing performed to estimate emotion information through machine learning prediction processing using an emotion learning model for each of two or more emotion information candidates. In other words, the flowchart in FIG. 26 shows processing performed to estimate emotion information through binary classification prediction processing in machine learning.

(Step S2601) The emotion estimation unit 336 acquires the two or more kinds of emotion source information acquired in step S1903.

(Step S2602) The emotion estimation unit 336 assigns 1 to a counter i.

(Step S2603) The emotion estimation unit 336 references the learning management unit 311 and judges whether or not an i^{th} emotion information candidate is present. If the i^{th} emotion information candidate is present, processing proceeds to step S2604, and otherwise processing proceeds to step S2609.

(Step S2604) The emotion estimation unit 336 acquires, from the learning management unit 311, the i^{th} emotion learning model paired with the i^{th} emotion information candidate.

(Step S2605) The emotion estimation unit 336 provides the two or more kinds of emotion source information acquired in step S2601 and the i^{th} emotion learning model acquired in step S2604 to a prediction processing module that performs binary classification in machine learning, and executes the module.

(Step S2606) The emotion estimation unit 336 judges whether or not the execution result in step S2605 is "true". If the execution result is "true", processing proceeds to step S2607, and if the execution result is "false", processing proceeds to step S2608.

(Step S2607) The emotion estimation unit 336 temporarily accumulates the score, which is part of the execution results in step S2605, in a buffer (not shown) in association with the i^{th} emotion information candidate.

(Step S2608) The emotion estimation unit 336 increments the counter i by one. Processing returns to step S2603.

(Step S2609) The emotion estimation unit 336 acquires the highest score and judges whether or not the score is not less than a threshold value. If the highest score is not less than the threshold value, processing proceeds to step S2610, and the highest score is less than the threshold value, processing proceeds to step S2611.

(Step S2610) The emotion estimation unit 336 acquires the emotion information paired with the highest score. Processing returns to the higher-level processing.

(Step S2611) The emotion estimation unit 336 acquires "empty" emotion information. Processing returns to the higher-level processing.

It should be noted that in the flowchart in FIG. 26, the processing from step S2608 to step S2611 do not necessarily have to be performed.

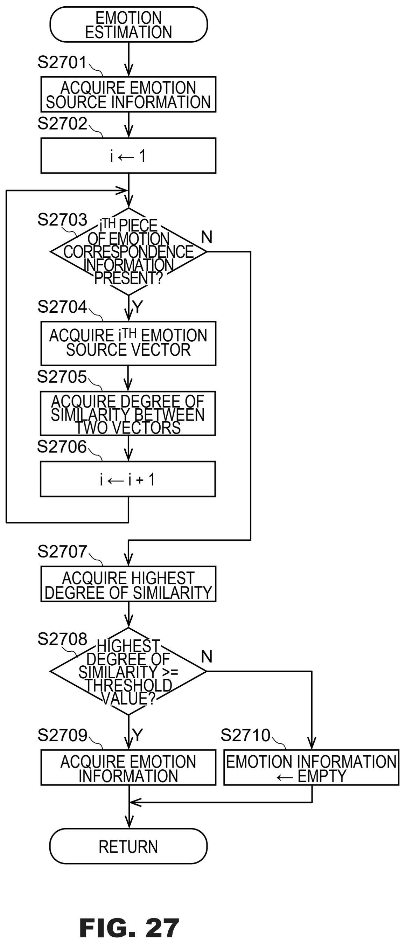

Next, an example of the third emotion estimation processing in step S1909 will be described with reference to the flowchart in FIG. 27. The flowchart in FIG. 27 shows processing performed to estimate emotion information using an emotion correspondence table.

(Step S2701) The emotion estimation unit 336 acquires the two or more kinds of emotion source information acquired in step S1903. The two or more kinds of emotion source information here are emotion source vectors.

(Step S2702) The emotion estimation unit 336 assigns 1 to a counter i.

(Step S2703) The emotion estimation unit 336 judges whether or not the i^{th} piece of emotion correspondence information is present in the emotion correspondence table in the learning management unit 311. If the i^{th} piece of emotion correspondence information is present, processing proceeds to step S2704, and otherwise processing proceeds to step S2707.

(Step S2704) The emotion estimation unit 336 acquires the i^{th} emotion source vector contained in the i^{th} piece of emotion correspondence information.

(Step S2705) The emotion estimation unit 336 acquires the degree of similarity between the emotion source vector acquired in step S2703 and the emotion source vector acquired in step S2704, and associates it with the i^{th} piece of emotion correspondence information.

(Step S2706) The emotion estimation unit 336 increments the counter i by one. Processing returns to step S2703.

(Step S2707) The emotion estimation unit 336 acquires the highest degree of similarity.

(Step S2708) The emotion estimation unit 336 judges whether or not the highest degree of similarity acquired in step S2707 is not less than a threshold value. If the highest degree of similarity is not less than the threshold value, processing proceeds to step S2709, and if the highest degree of similarity is less than the threshold value, processing proceeds to step S2710.

(Step S2709) The emotion estimation unit 336 acquires emotion information associated with the i^{th} piece of emotion correspondence information paired with the highest degree of similarity. Processing returns to the higher-level processing.

(Step S2710) The emotion estimation unit 336 acquires "empty" emotion information. Processing returns to the higher-level processing.

It should be noted that in the flowchart in FIG. 27, the processing from step S2708 to step S2711 do not necessarily have to be performed.

Next, an example of the output forming processing in step S1917 will be described with reference to the flowchart in FIG. 28.

(Step S2801) The forming unit 339 acquires a calendar template from the storage unit 31.

(Step S2802) The forming unit 339 assigns 1 to a counter i.

(Step S2803) The forming unit 339 judges whether or not an i^{th} time slot is present in the action management unit 313. If the i^{th} time slot is present, processing proceeds to step S2804, and otherwise processing returns to the higher-level processing. It should be noted that action information and emotion information are stored in the action management unit 313 in association with one or more time slots.

(Step S2804) The forming unit 339 judges whether or not the i^{th} time slot stored in the action management unit 313 is included in the period covered by the calendar template acquired in step S2801. If the i^{th} time slot is included, processing proceeds to step S2805, and if i^{th} time slot is not included, processing proceeds to step S2809.

(Step S2805) The forming unit 339 acquires the action information paired with the i^{th} time slot from the action management unit 313.

(Step S2806) The forming unit 339 acquires emotion information paired with the i^{th} time slot from the action management unit 313. It should be noted here that emotion information does not necessarily have to be successfully acquired.

(Step S2807) The forming unit 339 forms time slot information, which is information to be placed in the i^{th} time slot in the calendar, is information that can identify the action information acquired in step S2805, and is information that can identify the emotion information acquired in step S2806.

(Step S2808) The forming unit 339 places the time slot information formed in step S2807 at the position in the calendar specified by the i^{th} time slot.

(Step S2809) The forming unit 339 increments the counter i by one.

### Processing returns to step S2803.

Next, an example of the first action learning processing in step S1924 will be described with reference to the flowchart in FIG. 29. It is assumed that the action learning processing is performed by, for example, a learning unit (not shown). The learning unit may be a learning apparatus different from the action acquisition apparatus 3. The first action learning processing is processing performed to acquire a multi-value classification action learning model.

(Step S2901) The learning unit assigns 1 to a counter i.

(Step S2902) The learning unit judges whether or not the i^{th} piece of action information or the like is present in the action management unit 313. If the i^{th} piece of action information or the like is present, processing proceeds to step S2903, and otherwise processing proceeds to step S2905.

(Step S2903) Using the i^{th} piece of action information or the like, the learning unit forms action training data, and adds it to a buffer (not shown). The action training data is typically information that uses two or more kinds of action source information as illustrative variables and action information as an object variable.

(Step S2904) The learning unit increments the counter i by one. Processing returns to step S2902.

(Step S2905) The learning unit provides two or more pieces of action training data stored in a buffer (not shown) to a machine learning processing module, and executes the module to acquire an action learning model.

(Step S2906) The learning unit accumulates the action learning model acquired in step S2905 in the learning management unit 311.

It should be noted that in the flowchart in FIG. 29, instead of performing the learning processing in steps S2905 and S2906, the learning unit may accumulate, in the learning management unit 311, an action correspondence table containing two or more pieces of action training data as records (action correspondence information) in a buffer (not shown).

Next, an example of the second action learning processing in step S1924 will be described with reference to the flowchart in FIG. 30. The second action learning processing is processing performed to acquire a binary classification action learning model for each of two or more action information candidates.

(Step S3001) The learning unit assigns 1 to a counter i.

(Step S3002) The learning unit judges whether or not the i^{th} type of action information is present. If the i^{th} type of action information is present, processing proceeds to step S3003, and otherwise processing returns to the higher-level processing.

(Step S3003) The learning unit acquires the i^{th} type of action information.

(Step S3004) The learning unit acquires two or more positive examples, which are pieces of training data in the action management unit 313 and are pieces of action training that include the i^{th} type of action information.

(Step S3005) The learning unit acquires two or more negative examples, which are pieces of training data in the action management unit 313 and are pieces of action training data that do not include the i^{th} type of action information.

(Step S3006) The learning unit provides the two or more positive examples acquired in step S3004 and the two or more negative examples acquired in step S3005 to a machine learning processing module, and executes the module to acquires an action learning model.

(Step S3007) The learning unit accumulates, in the learning management unit 311, the action learning model acquired in step S3006, paired with the i^{th} type of action information.

(Step S3008) The learning unit increments the counter i by one. Processing returns to step S3002.

Next, an example of the first emotion learning processing in step S1925 will be described with reference to the flowchart in FIG. 31. It is assumed that the action learning processing is performed by, for example, a learning unit (not shown). The learning unit may be a learning apparatus different from the action acquisition apparatus 3. The first emotion learning processing is processing performed to acquire a multi-value classification emotion learning model.

(Step S3101) The learning unit assigns 1 to a counter i.

(Step S3102) The learning unit judges whether or not the i^{th} piece of emotion information or the like is present in the action management unit 313. If the i^{th} piece of emotion information or the like is present, processing proceeds to step S3103, and otherwise processing proceeds to step S3105.

(Step S3103) Using the i^{th} piece of emotion information or the like, the learning unit forms emotion training data, and adds it to a buffer (not shown). It should be noted that the emotion training data is information that uses two or more kinds of emotion source information as illustrative variables and emotion information as an object variable.

(Step S3104) The learning unit increments the counter i by one. Processing returns to step S3102.

(Step S3105) The learning unit provides two or more pieces of emotion training data stored in a buffer (not shown) to a machine learning processing module, and executes the module to acquire an emotion learning model.

(Step S3106) The learning unit accumulates the emotion learning model acquired in step S3105 in the learning management unit 311.

In the flowchart in FIG. 31, instead of performing the learning processing in steps S3105 and S3106, the learning unit may accumulate, in the learning management unit 311, an emotion correspondence table with two or more pieces of emotion training data as records (emotion correspondence information) in a buffer (not shown).

Next, an example of the second emotion learning processing in step S1925 will be described with reference to the flowchart in FIG. 32. The second emotion learning processing is processing performed to acquire a binary classification emotion learning model for each of two or more emotion information candidates.

(Step S3201) The learning unit assigns 1 to a counter i.

(Step S3202) The learning unit judges whether or not the i^{th} type of emotion information is present. If the i^{th} type of emotion information is present, processing proceeds to step S3203, and otherwise processing returns to the higher-level processing.

(Step S3203) The learning unit acquires the i^{th} type of emotion information.

(Step S3204) The learning unit acquires two or more positive examples, which are pieces of emotion training data in the action management unit 313 and which are pieces of emotion training data that include the i^{th} type of emotion information.

(Step S3205) The learning unit acquires two or more negative examples, which are pieces of emotion training data in the action management unit 313 and which are emotion training data that do not include the i^{th} type of emotion information.

(Step S3206) The learning unit provides the two or more positive examples acquired in step S3204 and the two or more negative examples acquired in step S3205 to a machine learning processing module, and executes the module to acquire an emotion learning model.

(Step S3207) The learning unit accumulates, in the learning management unit 311, the emotion learning model acquired in step S3206, paired with the i^{th} type of emotion information.

(Step S3208) The learning unit increments the counter i by one. Processing returns to step S3202.

Hereinafter, a specific example of the operation of the information system D according to the present embodiment will be described. Now, an action learning model acquired through machine learning processing using a large amount of training data including action source information of one or more users is stored in the storage unit 31 of the server apparatus 4. Also, an emotion learning model acquired through machine learning processing using a large amount of training data including emotion source information of one or more users is stored in the storage unit 31.

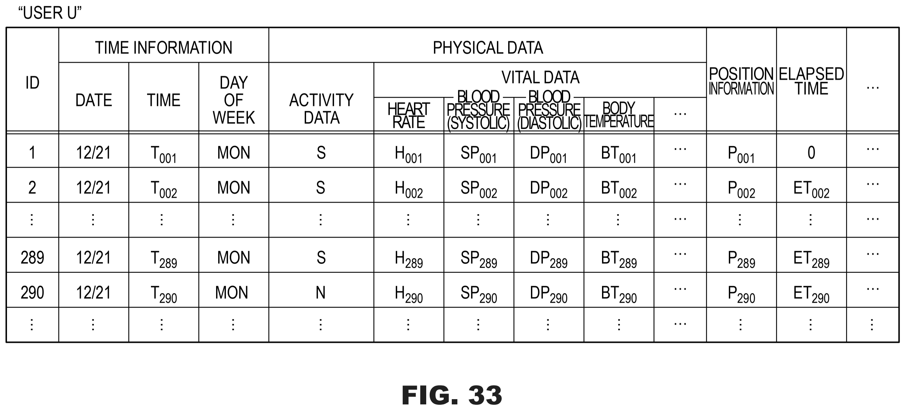

Also, in the storage unit 31 of the action acquisition apparatus 3, which is a terminal (for example, a smartwatch) held by a user "U₁", a large amount of action source information at two or more consecutive times acquired by the processing unit 33 are stored in an action source management table shown in FIG. 33 as a result of the above-described processing.

The action source management table (FIG. 33) is a set of records containing "ID", "time information", "physical data", "position information", and "elapsed time". "ID" is information that identifies a record. "Time information" is information that specifies a time, and here includes "date", "time", and "day of the week". "Physical data" includes "activity data" and "vital data". "Vital data" includes "heart rate", "blood pressure (systolic)", "blood pressure (diastolic)" and "body temperature". "Heart rate" is the number of heart beats per unit time (here, "1 minute"). "Blood pressure (systolic)" refers to the maximum blood pressure, and "blood pressure (diastolic)" refers to the minimum blood pressure. "Position information" is, for example, position information acquired through the processing described in the second embodiment. "Elapsed time" is the time that has elapsed since the same action started.

It is assumed that the user "U₁" inputs an output instruction to the action acquisition apparatus 3. Then, the action acquisition apparatus 3 accepts the output instruction.

Next, for example, the action estimation unit 335 accesses the server apparatus 4, receives the action learning model and the emotion learning model from the server apparatus 4, and accumulates the action learning model and the emotion learning model in the learning management unit 311.

Next, for example, the action estimation unit 335 forms an action source vector having time information, body data, position information, elapsed time, etc. for each record in FIG. 33 through the processing described with reference to the flowchart in FIG. 22. Next, the action estimation unit 335 acquires the action learning model from the learning management unit 311. Next, the action estimation unit 335 provides the action source vector and the action learning model to a machine learning prediction processing module, and executes the module. It is assumed that the action estimation unit 335 acquires action information "A₁" for the action source vectors from the record with "ID = 1" to the record with "ID = 289", and acquires action information "A₂" for the action source vectors from the record with "ID = 290" to the record with "ID = N". Then, for each record, the accumulation unit 338 accumulates the action information and the action confirmation flag "0" in the action management unit 313. It should be noted that "0" of the action confirmation flag and the emotion confirmation flag indicates that they have not yet been confirmed, and "1" indicates that they have been confirmed.

Also, for example, the emotion estimation unit 336 generates an emotion source vector having time information, physical data, position information, elapsed time, action information, etc. for each record in FIG. 33 through the processing described with reference to the flowchart in FIG. 25. Next, the emotion estimation unit 336 acquires an emotion learning model from the learning management unit 311. Next, the emotion estimation unit 336 provides the emotion source vector and the emotion learning model to a machine learning prediction processing module, and executes the module. It is assumed that the emotion estimation unit 336 acquires emotion information "E₁" for the action source vectors from the record with "ID = 1" to the record with "ID = 289", and acquires emotion information "E₂" for the action source vectors from the record with "ID = 290" to the record with "ID = N". Then, for each record, the accumulation unit 338 accumulates the emotion information and the emotion confirmation flag "0" in the action management unit 313.

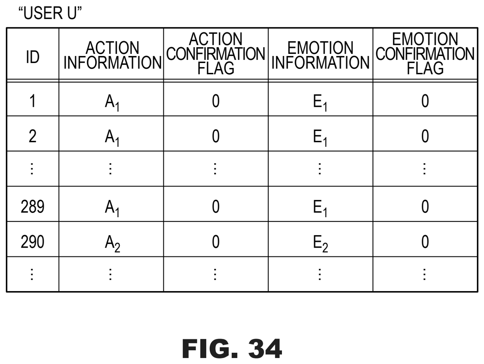

As a result of the above processing, the action and emotion management table shown in FIG. 34 is accumulated in the action management unit 313. The action and emotion management table contains two or more records each having "ID", "action information", "action confirmation flag", "emotion information", and "emotion confirmation flag".

Next, for each record in FIGS. 33 and 34, the accumulation unit 338 acquires the time slot (e.g., "TZ₁", which is "T₀₀₁ to T₂₈₉") corresponding to the time (e.g., T₀₀₁, ..., T₀₀₂, T₂₈₉) paired with the same action information (e.g., "A₁"), and accumulates the time slot and the action information in association with each other.

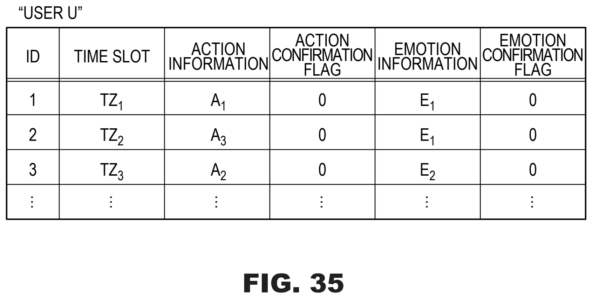

Also, for each record in FIGS. 33 and 34, the accumulation unit 338 acquires the time slot (e.g., "TZ₁", which is "T₀₀₁ to T₂₈₉") corresponding to the time (e.g., T₀₀₁, ..., T₀₀₂, T₂₈₉) paired with the same emotion information (e.g., "E₁"), and accumulates the time slot and the emotion information in association with each other. It should be noted that, at this stage, the action confirmation flag and emotion confirmation flag corresponding to the time slots are both "0". An example of the accumulated information is shown in FIG. 35. FIG. 35 is a time slot information management table. The time slot information management table contains one or more records each having an "ID", "time slot", "action information", "action confirmation flag", "emotion information", and "emotion confirmation flag".

Then, the forming unit 339 performs the processing described with reference to the flowchart in FIG. 28 using one or more sets of a time slot, action information, and emotion information accumulated by the accumulation unit 338, forms time slot information for each piece of action information, arranges it in the calendar template, and forms output information.

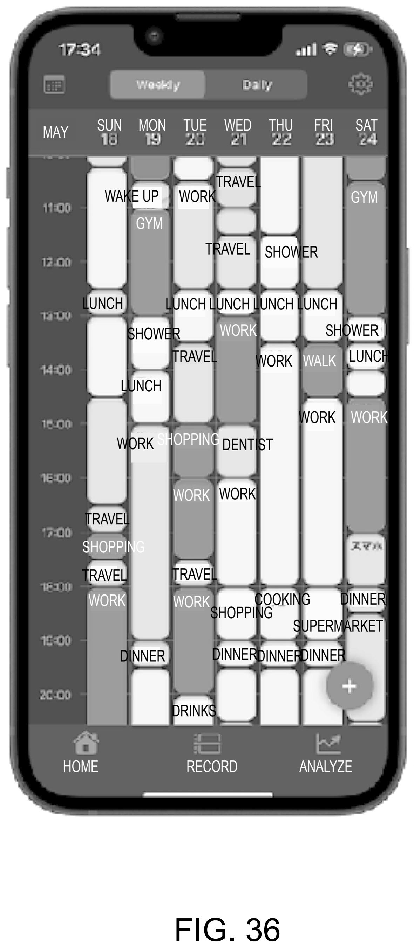

Next, the output unit 34 outputs the output information. An example of such an output is shown in FIG. 36. In FIG. 36, estimated action information for each time slot on each day is displayed on a calendar.

If the action information for each time slot is correct, the user "U₁" inputs a "confirmation instruction" for the displayed action information, and if the estimated action information is incorrect, inputs the correct action information. Also, if the emotion information for each time slot is correct, the user "U₁" inputs a "confirmation instruction" for the out emotion information, and if the estimated emotion information is incorrect, the user inputs the correct emotion information. In response to the user's input, the "action information", "action confirmation flag", "emotion information," and "emotion confirmation flag" in FIG. 35 will be changed.

As described above, according to the present embodiment, the user's action can be estimated using position information corresponding to a time.

Also, according to the present embodiment, the user's action can be estimated using position information corresponding to a time and the user's activity data corresponding to the time.

Also, according to the present embodiment, the user's action can be estimated with higher accuracy using position information corresponding to a time, the user's activity data corresponding to the time, and the user's vital data corresponding to the time.

Also, according to the present embodiment, the user's emotions during the action can be estimated.

Also, according to the present embodiment, the user's action can be estimated with higher accuracy using past records.

Also, according to the present embodiment, the users' action can be estimated with higher accuracy, using the past records of two or more users.

Also, according to the present embodiment, when the user's action cannot be estimated, the place where the user has been present can be output.

Furthermore, according to the present embodiment, it is possible to estimate user's action with high accuracy even in places where GPS signals cannot be received, by using indoor position information or the like acquired by the method for acquiring position information, described in the second embodiment.

It should be noted that the processing in this embodiment may be realized using software. This software may be distributed through software downloading or the like. Also, this software may be recorded on a recording medium such as a CD-ROM and distributed. It should be noted that the same applies to the other embodiments in the present description. The software that realizes the information system D according to the present embodiment is the program described below. That is to say, this program is a program that enables a computer to function as: a time acquisition unit configured to acquire a time; a position acquisition unit configured to acquire position information associated with the time; an action estimation unit configured to, using two or more pieces of action source information including the position information associated with the time, acquire action information that specifies an action of a user during a time slot specified by the times contained in the two or more pieces of action source information; and an action output unit configured to output the action information corresponding to the time slot.

### Fourth Embodiment

The differences between the present embodiment and the third embodiment are as follows. That is to say, in the present embodiment, the action acquisition apparatus is a server, and the action acquisition apparatus estimates the action information and emotion information of the user using the position information and the like received from the user's terminal apparatus.

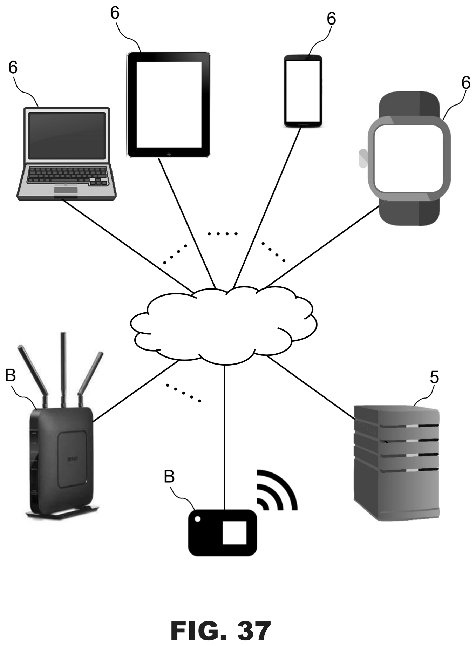

FIG. 37 is a conceptual diagram of an information system E according to the present embodiment. The information system E includes an action acquisition apparatus 5, one or more terminal apparatuses 6, and one or more communication apparatuses B.

Each action acquisition apparatus 5 is a server, and is, for example, a cloud server or an ASP server, but there is no limitation on the type thereof. The action acquisition apparatus 5 is an apparatus that receives action source information such as position information from the user's terminal apparatus 6, estimates the user's action information using the action source information, and transmits the action information to the terminal apparatus 6. The action acquisition apparatus 5 is an apparatus that receives emotion source information from the user's terminal apparatus 6, estimates the user's emotion information using the emotion source information, and transmits the action information to the terminal apparatus 6.

The terminal apparatus 6 is a terminal used by a user. The terminal apparatus 6 is, for example, a smartphone, a tablet terminal, a smart watch, a so-called personal computer, or the like, and there is no limitation on the type thereof. The terminal apparatus 6 is a terminal that transmits action source information and emotion source information including position information and the like to the action acquisition apparatus 5, and receives and outputs action information and emotion information from the action acquisition apparatus 5. The apparatus that transmits the action source information and the emotion source information to the action acquisition apparatus 5 may be a different apparatus from the apparatus that receives the action information and the emotion information from the action acquisition apparatus 5 and outputs them.

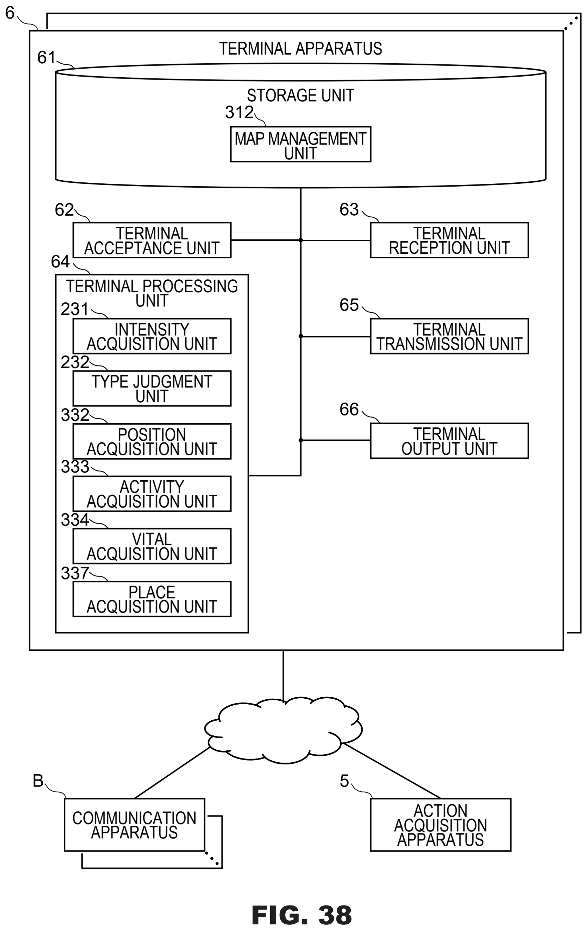

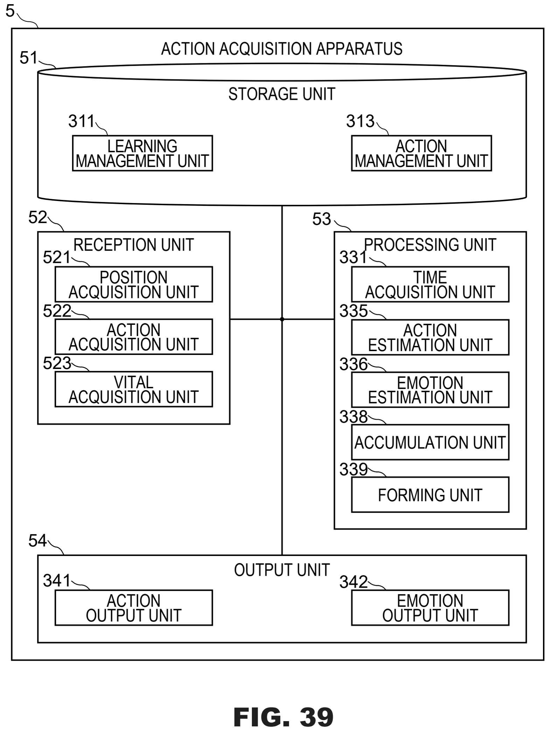

FIG. 38 is a block diagram of the information system E according to the present embodiment. FIG. 39 is a block diagram of the action acquisition apparatus 5.

The action acquisition apparatus 5 includes a storage unit 51, a reception unit 52, a processing unit 53, and a transmitting unit 54. The storage unit 51 includes the learning management unit 311 and the action management unit 313. The reception unit 52 includes a position acquisition unit 521, an activity acquisition unit 522, and a vital acquisition unit 523. The processing unit 53 includes the time acquisition unit 331, the action estimation unit 335, the emotion estimation unit 336, the accumulation unit 338, and the forming unit 339. The transmitting unit 54 includes the action output unit 341 and the emotion output unit 342.

The terminal apparatus 6 includes a terminal storage unit 61, a terminal acceptance unit 62, a terminal reception unit 63, a terminal processing unit 64, a terminal transmitting unit 65, and a terminal output unit 66. The terminal storage unit 61 includes the map management unit 312. The terminal processing unit 64 includes the intensity acquisition unit 231, the type judgment unit 232, the position acquisition unit 332, the activity acquisition unit 333, the vital acquisition unit 334, and the place acquisition unit 337.

The storage unit 51 included in the action acquisition apparatus 5 stores various kinds of information. Examples of the various kinds of information include the learning information described above and the action information described above.

The reception unit 52 receives various kinds of instructions and information from the terminal apparatus 6. Examples of the various kinds of instructions and information include position information, activity data, vital data, an output instruction, a confirmation instruction, action information to be corrected, and emotion information to be corrected.

It is preferable that the reception unit 52 receives position information, activity data, vital data, and so on from the terminal apparatus 6 all at once. It is preferable that the reception unit 52 receives the position information and the like associated with the user identifier all at once. The user identifier is information that identifies the user who uses the terminal apparatus 6. The user identifier is, for example, a user ID, a telephone number, an email address, or an identifier of the terminal apparatus 6. The identifier of the terminal apparatus 6 is, for example, an IP address.

The position acquisition unit 521 receives position information from the terminal apparatus 6. Such position information is associated with time. It is preferable that the position acquisition unit 521, upon receiving position information, acquires the time from a clock (not shown) and associates the time with the position information. It is preferable that such position information is associated with a user identifier.

The activity acquisition unit 522 receives activity data from the terminal apparatus 6. Such activity data is associated with time. It is preferable that the activity acquisition unit 522, upon receiving activity data, acquires the time from a clock (not shown) and associates the time with the activity data. It is preferable that such activity data is associated with a user identifier.

The vital acquisition unit 523 receives one or more types of vital data from the terminal apparatus 6. Such vital data is associated with time. It is preferable that the vital acquisition unit 523, upon receiving vital data, acquires the time from a clock (not shown) and associates the time with the vital data. It is preferable that such vital data is associated with a user identifier.

The processing unit 53 performs various kinds of processing. The various kinds of processing are, for example, processing performed by the time acquisition unit 331, the action estimation unit 335, the emotion estimation unit 336, and the accumulation unit 338.

The transmitting unit 54 transmits various kinds of information to the terminal apparatus 6. Examples of the various kinds of information include estimated action information, estimated emotion information, and output information formed by the forming unit 339.

The action output unit 341 transmits the action information acquired by the action estimation unit 335 and associated with a time slot to the terminal apparatus 6.

The emotion output unit 342 transmits, to the terminal apparatus 6, the emotion information acquired by the emotion estimation unit 336 and associated with a time slot.

The terminal storage unit 61 included in the terminal apparatus 6 stores various kinds of information. Examples of the various kinds of information include position information, activity data, and vital data.

The terminal acceptance unit 62 accepts various kinds of instructions and information. Examples of the various instructions and information include an output instruction, a confirmation instruction, action information modified by the user based on the estimated action information, and emotion information modified by the user based on the estimated emotion information.

Any input means, such as a touch panel, a keyboard, a mouse, or a menu screen, may be used to input the various kinds of instructions and information.

The terminal reception unit 63 receives various kinds of information from the action acquisition apparatus 5. Examples of the various kinds of information include output information, action information, and emotion information.

The terminal processing unit 64 performs various kinds of processing. Examples of the various kinds of processing include processing performed to change the instructions, information, and so on accepted by the terminal acceptance unit 62 into instructions, information, and so on having a structure suitable for transmission, and processing performed to change the information received by the terminal reception unit 63 into a structure suitable for output.

The terminal transmitting unit 65 transmits various kinds of instructions and information. Examples of the various kinds of instructions and information include an output instruction, a confirmation instruction, action information to be changed, and emotion information to be changed.

The terminal output unit 66 outputs various kinds of information. Examples of the various kinds of information include output information, action information, and emotion information.

The storage unit 51 and the terminal storage unit 61 are preferably nonvolatile recording media, but they can also be realized using volatile recording media.

There is no limitation on the process in which information is stored in the storage unit 51 or the like. For example, information may be stored in the storage unit 51 or the like via a recording medium, or information transmitted via a communication line or the like may be stored in the storage unit 51 or the like, or information input via an input device may be stored in the storage unit 51 or the like.

The reception unit 52, the position acquisition unit 521, the activity acquisition unit 522, the vital acquisition unit 523, the transmitting unit 54, the action output unit 341, the emotion output unit 342, the terminal reception unit 63, and the terminal transmitting unit 65 are typically realized using wireless or wired communication means.

The processing unit 53 and the terminal processing unit 64 can typically be realized using a processor, a memory, and so on. The processing procedures performed by the processing unit 53 and so on are typically realized using software, and the software is recorded on a recording medium such as a ROM. However, such processing procedures may be realized using hardware (a dedicated circuit). It should be noted that the processor may be a CPU, an MPU, a GPU, or the like, and there is no limitation on the type thereof.

The terminal acceptance unit 62 can be realized using, for example, a device driver for the input means such as a touch panel or a keyboard, or control software or the like for controlling the menu screen.

The terminal output unit 66 may or may not be considered to include an output device such as a display or a speaker. The terminal output unit 66 can be realized using the driver software of the output device, the driver software of the output device and the output device, or the like.

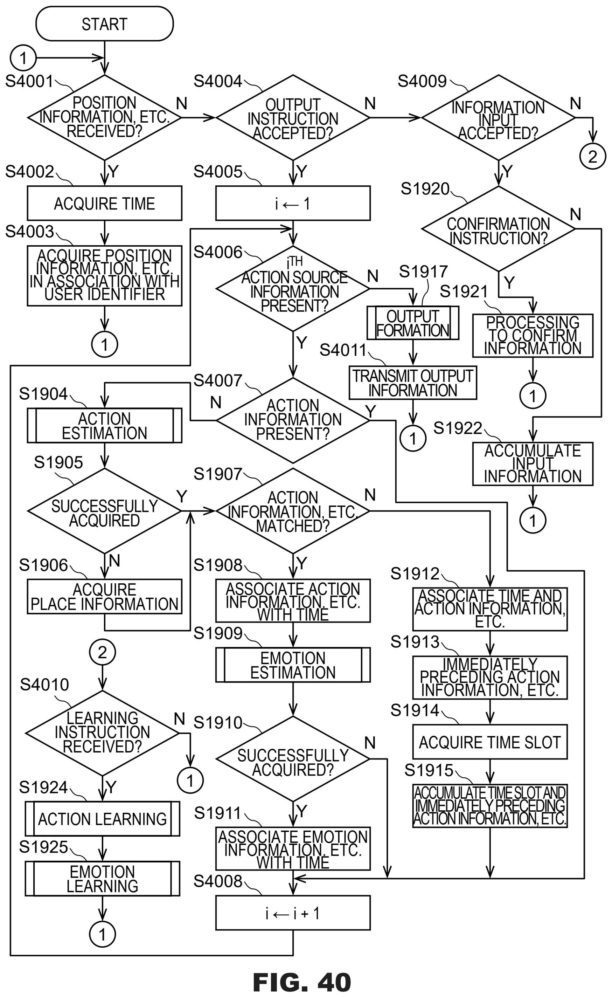

Next, an example of the operation of the action acquisition apparatus 5 will be described with reference to the flowchart in FIG. 40. In the flowchart in FIG. 40, the description of the same steps as those in FIG. 19 will be omitted.

(Step S4001) The reception unit 52 judges whether or not position information, etc. paired with a user identifier has been received from the terminal apparatus 6. If position information, etc. has been received, processing proceeds to step S4002, and otherwise processing proceeds to step S4004. It should be noted that the position information, etc., is, for example, a user identifier and position information. The position information, etc. is, for example, a user identifier and position information, and one or more kinds of information among activity data and vital data.

(Step S4002) The time acquisition unit 331 acquires the time from a clock (not shown). Here, the time acquisition unit 331 may also acquire the day of the week. The time typically includes the hour and minute. The time may include one or more pieces of information selected from the year, month, and day.

(Step S4003) The accumulation unit 338 accumulates the position information, etc. and the time received in step S4001 in the action management unit 313 in association with the user identifier. Processing returns to step S4001.

(Step S4004) The reception unit 52 judges whether or not an output instruction has been received from the terminal apparatus 6. If an output instruction has been received, processing proceeds to step S4005, and otherwise processing proceeds to step S4009. It should be noted that the output instruction to be received is typically associated with a user identifier. In addition, the output instruction typically includes period information that specifies the period for which the action information is to be acquired (e.g., "from December 17, 2023 to December 23, 2023").

(Step S4005) The action estimation unit 335 assigns 1 to a counter i.

(Step S4006) The action estimation unit 335 judges whether or not the i^{th} piece of action source information paired with the user identifier associated with the output instruction received in step S4004 is present in the action management unit 313. If the i^{th} piece of action source information is present, processing proceeds to step S4007, and otherwise processing proceeds to step S1917.

(Step S4007) The action estimation unit 335 judges whether or not action information associated with the i^{th} piece of action source information is present. If the action information is present, processing proceeds to step S4008, and otherwise processing proceeds to step S1904. It should be noted that when action information associated with action source information is present, it typically means that the action information has already been estimated using the action source information.

(Step S4008) The action estimation unit 335 increments the counter i by one. Processing returns to step S4006.

(Step S4009) The reception unit 52 judges whether or not information, etc. has been received from the terminal apparatus 6. If information etc. has been received, processing proceeds to step S1920, and otherwise processing proceeds to step S4010. Examples of the information, etc. include a confirmation instruction, action information to be corrected, and emotion information to be corrected.

(Step S4010) The reception unit 52 judges whether or not a learning instruction has been received from the terminal apparatus 6. If a learning instruction has been received, processing proceeds to step S1924, and otherwise processing returns to step S4001.

(Step S4011) The transmitting unit 54 transmits the output information formed in step S1917 to the terminal apparatus 6.

### Processing returns to step S4001.

In the flowchart in FIG. 40, processing is terminated when power is turned off or an interruption is made to terminate the processing.

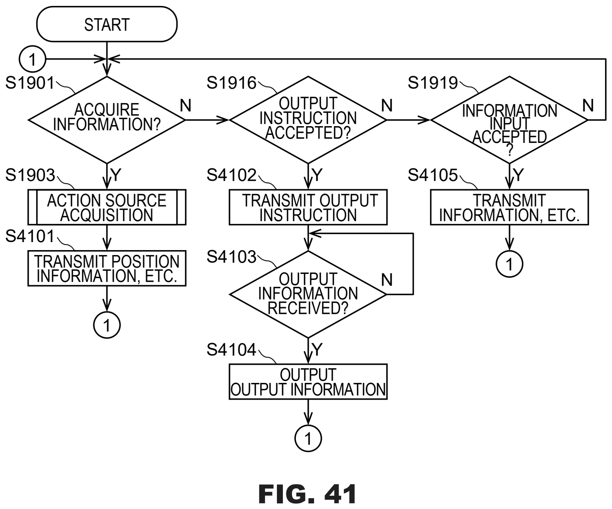

Next, an example of the operation of the terminal apparatus 6 will be described with reference to the flowchart in FIG. 41. In the flowchart in FIG. 41, the description of the same steps as those in FIG. 19 will be omitted.

(Step S4101) The terminal transmitting unit 65 acquires the user identifier from the terminal storage unit 61, and transmits the position information and so on acquired in step S1903 to the action acquisition apparatus 5 in association with the user identifier.

(Step S4102) The terminal transmitting unit 65 transmits the output instruction accepted in step S1916 to the action acquisition apparatus 5 in association with the user identifier from the terminal storage unit 61. It should be noted that the output instruction typically includes period information.

(Step S4103) The terminal reception unit 63 judges whether or not output information has been received from the action acquisition apparatus 5. If output information has been received, processing proceeds to step S4104, and otherwise processing returns to step S4103.

(Step S4104) Using the received output information, the terminal processing unit 64 forms output information to be output. The terminal output unit 66 outputs the output information. Processing returns to step S1901.

(Step S4105) The terminal transmitting unit 65 transmits, to the action acquisition apparatus 5, the information, etc. acquired from the information input accepted in step S1919, in association with the user identifier from the terminal storage unit 61. Processing returns to step S1901.

It should be noted that examples of the information, etc. include a confirmation instruction, changed action information, or changed emotion information. It should be noted that the confirmation instruction includes information that specifies the action information to be confirmed or the emotion information to be confirmed. The changed action information is associated with information that identifies the action information to be corrected. The changed emotion information is associated with information that specifies the emotion information to be corrected.

It should be noted that in the flowchart in FIG. 41, processing is terminated when power is turned off or an interruption is made to terminate the processing.

As described above, according to the present embodiment, the user's action can be estimated using position information corresponding to a time.

Also, according to the present embodiment, the user's action can be estimated using position information corresponding to a time and the user's activity data corresponding to the time.

Also, according to the present embodiment, the user's action can be estimated with higher accuracy using position information corresponding to a time, the user's activity data corresponding to the time, and the user's vital data corresponding to the time.

Also, according to the present embodiment, the user's emotions during the action can be estimated.

Also, according to the present embodiment, the user's action can be estimated with higher accuracy using past records.

Also, according to the present embodiment, the users' action can be estimated with higher accuracy, using the past records of two or more users.

Also, according to the present embodiment, when the user's action cannot be estimated, the place where the user has been present can be output.

Furthermore, the software that realizes the action acquisition apparatus 5 according to the present embodiment is the program described below. That is to say, this program is a program that enables a computer to function as: a time acquisition unit configured to acquire a time; a position acquisition unit configured to acquire position information associated with the time; an action estimation unit configured to, using two or more pieces of action source information including the position information associated with the time, acquire action information that specifies an action of a user during a time slot specified by the time contained in the two or more pieces of action source information; and an action output unit configured to output the action information corresponding to the time slot.

### Fifth Embodiment

The present embodiment describes an information processing apparatus that detects an action set, which is a series of actions, from a group of action information corresponding to the user's time slots, and acquires and outputs keystone habit information containing action information that serves as a starting point of the action set.

The present embodiment describes an information processing apparatus that also acquires and outputs a start time of the keystone habit.

The present embodiment describes an information processing apparatus that also uses emotion information to detect the action set.

The present embodiment describes an information processing apparatus that acquires and outputs keystone habit information corresponding to a query from a user.

Furthermore, the present embodiment describes an information processing apparatus that provides a user with a recommendation related to an action by using the acquired keystone habit information.

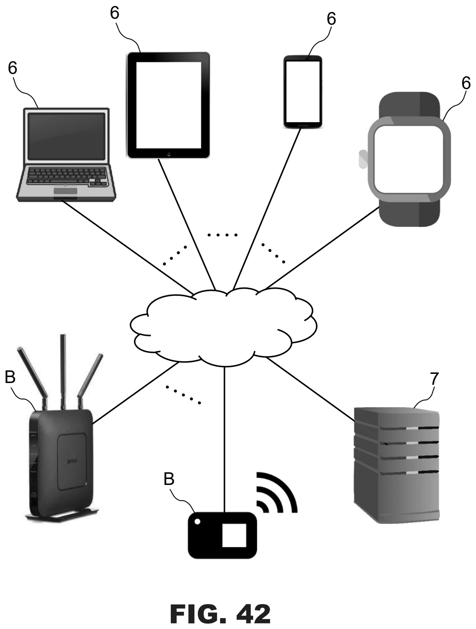

FIG. 42 is a conceptual diagram of an information system F according to the present embodiment. The information system F includes an information processing apparatus 7, one or more terminal apparatuses 6, and three or more communication apparatuses B.

The information processing apparatus 7 is a server that transmits keystone habit information to the terminal apparatuses 6. The information processing apparatus 7 is, for example, a cloud server or an ASP server, but there is no limitation on the type thereof. It is preferable that the information processing apparatus 7 has the functions of the server apparatus 4 and the action acquisition apparatus 5.

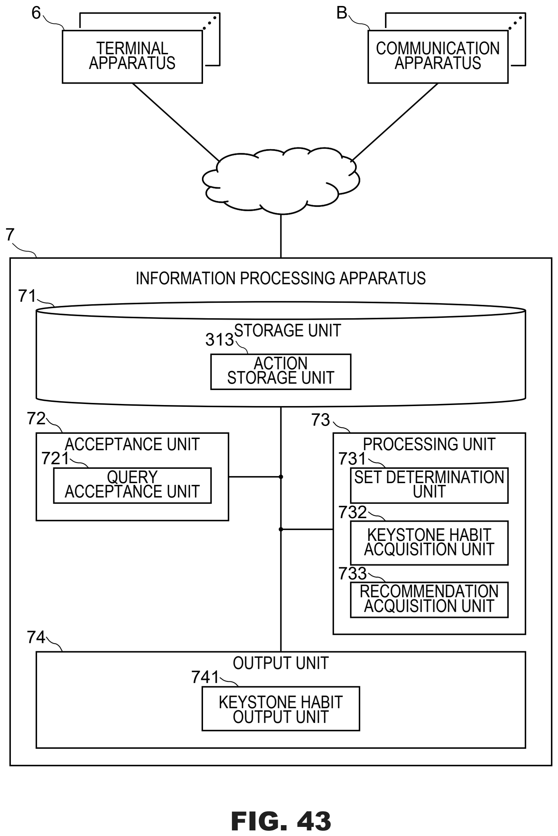

FIG. 43 is a block diagram of the information system F according to the present embodiment. The information processing apparatus 7 includes a storage unit 71, an acceptance unit 72, a processing unit 73, and an output unit 74. The storage unit 71 includes an action management unit 313. The acceptance unit 72 includes a query acceptance unit 721. The processing unit 73 includes a set determination unit 731, a keystone habit acquisition unit 732, and a recommendation acquisition unit 733. The output unit 74 includes a keystone habit output unit 741.

Various types of information are stored in the storage unit 71 included in the information processing apparatus 7. Examples of the various kinds of information include learning information, action information, and one or more recommendation templates.

Recommendation templates are pieces of information serving as templates for forming recommendations. Each recommendation template is, for example, a positive recommendation template or a negative recommendation template. A positive recommendation template is template information for recommending a keystone habit and includes, for example, a sentence such as: "During the period specified by <time slot>, the keystone habit, which is <action information>, should be performed". In the template, <time slot> and <action information> are variables, and the time slot of the keystone habit and the action information that is the keystone habit are substituted therein. A negative recommendation template is template information for discouraging a keystone habit and includes, for example, a sentence such as: "During the period specified by <time slot>, the keystone habit, which is <action information>, should be stopped".

The action management unit 313 stores pieces of action information corresponding to four or more time slots. It is preferable that each of the four or more pieces of action information be associated with a user identifier. It is preferable that the action management unit 313 stores action information, emotion information, and so on acquired by the action acquisition apparatus 5. However, the action management unit 313 may store action information, emotion information, and so on that have been input manually. There is no limitation on the process in which action information, emotion information, and so on are stored in the action management unit 313.

The acceptance unit 72 accepts various kinds of instructions and information. Examples of the various kinds of instructions and information include an output instruction and a query.

An output instruction is an instruction to output keystone habit information. An output instruction is typically associated with a user identifier.

The "acceptance" here is typically reception of information transmitted via a wired or wireless communication network, but may be a concept that includes, for example, acceptance of information input from an input device such as a keyboard, a mouse, or a touch panel, and acceptance of information read out from a recording medium such as an optical disk, a magnetic disk, or a semiconductor memory.

The query acceptance unit 721 accepts a query. A query may also be referred to as an inquiry. A query includes a time and action information. A query may also include emotion information. Examples of a query include "<time> 7:00 <action information> waking up" and "<time> 7:00 <action information> waking up <emotion information> positive". The query "<time> 7:00 <action information> waking up" is an inquiry requesting a keystone habit for waking up at 7 a.m. The query "<time> 7:00 <action information> waking up <emotion information> positive" is an inquiry requesting a keystone habit for waking up comfortably at 7 a.m.

The processing unit 73 performs various kinds of processing. An example of the various kinds of processing is the processing performed by the set determination unit 731, the keystone habit acquisition unit 732, or the recommendation acquisition unit 733.

The set determination unit 731 references the action management unit 313 and determines an action set that satisfies a set condition. The set determination unit 731 determines an action set for each user. It is preferable that the set determination unit 731 determine the action set by using emotion information. Note that the action management unit 313 may be provided in an external apparatus (not shown).

An action set is a set of two or more pieces of action information that are temporally continuous. An action set is a set of action information of a user.

A set condition is information for determining an action set. A set condition is a condition for judging the presence of an action set. The set condition is typically a condition regarding the number of occurrences of an action set within a group of action information of a user. For example, the set condition is that the action set appears N times or more (where N is a natural number greater than or equal to 2) in the action management unit 313.

The keystone habit acquisition unit 732 acquires from the action management unit 313 keystone habit information including action information specifying an action that serves as a starting point of the action set determined by the set determination unit 731. The action information specifying an action that serves as a starting point of an action set is the action information regarding the initial time slot contained in the action set.

For example, the keystone habit acquisition unit 732 acquires, from the action management unit 313, keystone habit information containing action information specifying the action serving as the starting point of the action set determined by the set determination unit 731, and the start time included in the time slot corresponding to the action information.

For example, the keystone habit acquisition unit 732 acquires a time slot and action information corresponding to the time and the action information contained in an accepted query and acquires keystone habit information including action information specifying an action that serves as the starting point of the action set in the time slot that precedes the acquired time slot and is closest to the determined time slot. It is preferable that the keystone habit information include a start time or a time slot of the starting-point action information. The keystone habit information may contain emotion information paired with the starting-point action information.

The recommendation acquisition unit 733 forms a recommendation including the keystone habit information acquired by the keystone habit acquisition unit 732.

For example, the recommendation acquisition unit 733 forms a recommendation by substituting the keystone habit information into a recommendation template stored in the storage unit 71.

For example, the recommendation acquisition unit 733 acquires emotion information corresponding to the action information contained in the keystone habit information or the action information contained in the query and forms a recommendation by using the emotion information and the keystone habit information.

For example, the recommendation acquisition unit 733 acquires, from the storage unit 71, a recommendation template corresponding to the emotion information, substitutes the keystone habit information into the recommendation template, and forms the recommendation.

The output unit 74 outputs various kinds of information. Examples of the various kinds of information include a keystone habit, a start time, a time slot, and a recommendation.

The keystone habit output unit 741 outputs the keystone habit information acquired by the keystone habit acquisition unit 732. The keystone habit output unit 741 outputs the recommendation acquired by the recommendation acquisition unit 733.

The "output" here typically means transmission to a terminal apparatus 6, but may be a concept that encompasses displaying on a display screen, projection using a projector, printing by a printer, the output of a sound, transmission to an external apparatus, accumulation on a recording medium, delivery of a processing result to another processing apparatus or another program, and the like.

It is preferable that the storage unit 71 is a nonvolatile recording medium, but it can be realized using a volatile recording medium.

There is no limitation on the process in which information is stored in the storage unit 71. For example, information may be stored in the storage unit 71 or the like via a recording medium, or information transmitted via a communication line or the like may be stored in the storage unit 71 or the like, or information input via an input device may be stored in the storage unit 71 or the like.

It is preferable that the acceptance unit 72 and the query acceptance unit 721 are realized using wireless or wired communication means, but they may also be realized using, for example, means for receiving broadcast, a device driver for the input means such as a touch panel or a keyboard, or control software or the like for controlling the menu screen.

The processing unit 73, the set determination unit 731, the keystone habit acquisition unit 732, and the recommendation acquisition unit 733 are typically realized using a processor, a memory, and so on. The processing procedures performed by the processing unit 73 and so on are typically realized using software, and the software is recorded on a recording medium such as a ROM. However, such processing procedures may be realized using hardware (a dedicated circuit). Note that the processor is a CPU, an MPU, a GPU, or the like, and there is no limitation on the type thereof.

It is preferable that the output unit 74 and the keystone habit output unit 741 are realized using wireless or wired communication means, but they may be realized using, for example, the driver software of an output device such as a display or a speaker, the driver software of the output device and the output device, or the like.

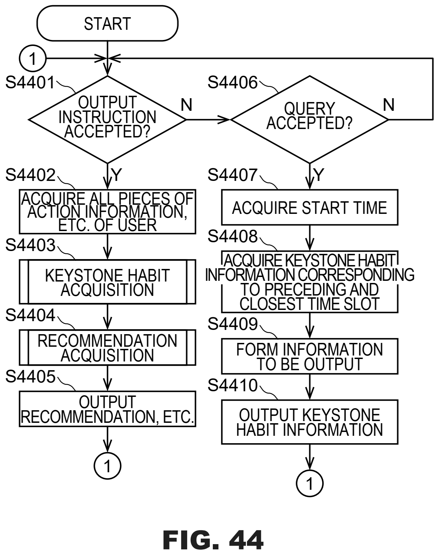

Next, the operation example of the information processing apparatus 7 will be described with reference to the flowchart of FIG. 44.

(Step S4401) The acceptance unit 72 judges whether or not an output instruction has been accepted. If an output instruction has been accepted, processing proceeds to step S4402, and otherwise processing proceeds to step S4406. Here, for example, the acceptance unit 72 judges whether or not an output instruction has been received from a terminal apparatus 6.

(Step S4402) The processing unit 73 acquires a user identifier corresponding to the output instruction accepted in step S4401. Next, the processing unit 73 acquires, from the action management unit 313, all pieces of action information and so on paired with the user identifier. The action information and so on include action information and time slots. It is preferable that the action information and so on include emotion information. It is assumed that the acquired action information has been sorted in order of the time slots.

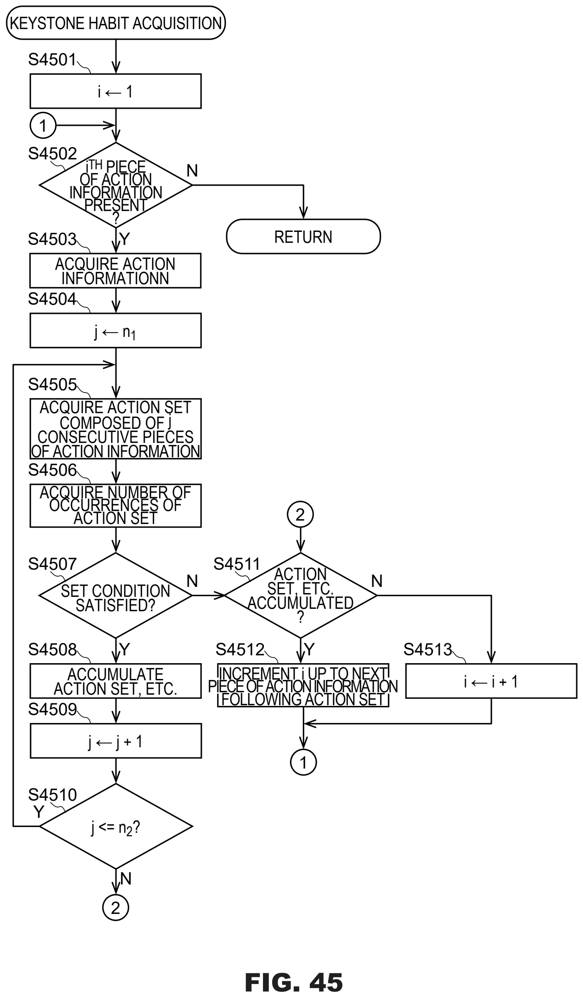

(Step S4403) The processing unit 73 performs a keystone habit acquisition processing, using the action information and so on acquired in step S4402. An example of keystone habit acquisition processing will be described with reference to the flowchart in FIG. 45.

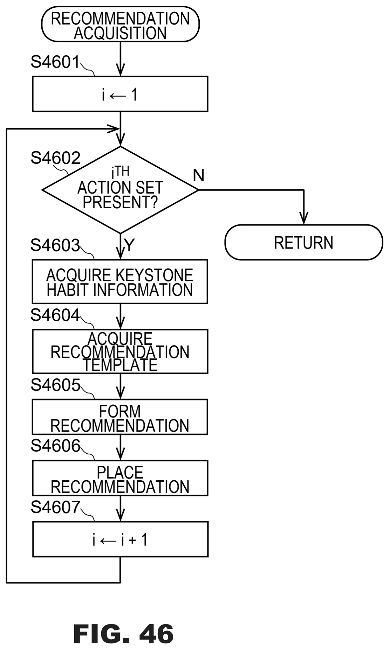

(Step S4404) The recommendation acquisition unit 733 performs recommendation acquisition processing. An example of recommendation acquisition processing will be described with reference to the flowchart in FIG. 46.

(Step S4405) The keystone habit output unit 741 outputs a recommendation and the like. Processing returns to step S4401. The recommendation and so on is, for example, one or more recommendations and one or more pieces of keystone habit information. Here, for example, the keystone habit output unit 741 transmits a recommendation and so on to the terminal apparatus 6.

(Step S4406) The query acceptance unit 721 judges whether or not a query has been accepted. If a query has been accepted, processing proceeds to step S4407, and otherwise processing returns to step S4401. Here, for example, the query acceptance unit 721 judges whether or not a query has been acquired from a terminal apparatus 6.

(Step S4407) The keystone habit acquisition unit 732 acquires a start time included in the query.

(Step S4408) The keystone habit acquisition unit 732 acquires, from the action management unit 313, keystone habit information corresponding to the time slot that precedes and is closest to the start time.

(Step S4409) The processing unit 73 forms information to be output, which includes the keystone habit information acquired in step S4408.

(Step S4410) The keystone habit output unit 741 outputs the information formed in step S4409. Processing returns to step S4401. Here, for example, the keystone habit output unit 741 transmits the formed information to the terminal apparatus 6.

In the flowchart in FIG. 44, there is no limitation on the trigger for acquiring and accumulating an action set or keystone habit information.

In the flowchart in FIG. 44, processing is terminated when power is turned off or an interruption is made to terminate the processing.

Next, an example of the keystone habit acquisition processing in step S4403 will be described with reference to the flowchart in FIG. 45.

(Step S4501) The set determination unit 731 substitutes 1 for a counter i.

(Step S4502) The set determination unit 731 judges whether or not the i^{th} piece of action information acquired in step S4402 is present. If the i^{th} piece of action information is present, processing proceeds to step S4503, and otherwise processing returns to the higher-level processing.

(Step S4503) The set determination unit 731 acquires the i^{th} piece of action information acquired in step S4402.

(Step S4504) The set determination unit 731 substitutes n₁ for a counter j. Here, n₁ is the minimum number of pieces of action information that can constitute an action set, for example, "2," "3," or "4," but there is no limitation.

(Step S4505) The set determination unit 731 acquires, from among the group of action information acquired in step S4402, an action set composed of j consecutive pieces of action information starting from the i^{th} piece of action information.

(Step S4506) The set determination unit 731 acquires the number of occurrences of the action set acquired in step S4505 among the group of action information acquired in step S4402.

(Step S4507) The set determination unit 731 judges whether or not the number of occurrences acquired in step S4506 satisfies the set condition. If the set condition is satisfied, processing proceeds to step S4508, and otherwise processing proceeds to step S4511.

(Step S4508) The keystone habit acquisition unit 732 accumulates, in the storage unit 71, the action set and so on acquired in step S4505. The action set and so on may be a group of pairs of action information and a start time, or a group of pairs of action information and a time slot. It is preferable that the action set and so on contain emotion information associated with action information. The keystone habit acquisition unit 732 may accumulate, separately from the action set and in association with the action set, keystone habit information including action information serving as a starting point of the action set, in the storage unit 71.

(Step S4509) The set determination unit 731 increments the counter i by one.

(Step S4510) The set determination unit 731 judges whether or not the counter j is not greater than n₂. If the counter j is not greater than n₂, processing returns to step S4504, and if the counter j is greater than n₂, processing proceeds to step S4511. Here, n₂ is the maximum number of pieces of action information that can constitute an action set, for example, "6," "7," or "8," but there is no limitation.

(Step S4511) The set determination unit 731 judges whether or not the action set and so on starting from the i^{th} piece of action information have been accumulated. If the action set and so on have been accumulated, processing proceeds to step S4512, and otherwise processing proceeds to step S4513.

(Step S4512) The set determination unit 731 increments the counter i by one up to the piece of action information following the last piece of action information contained in the stored action set. Processing returns to step S4502.

(Step S4513) The set determination unit 731 increments the counter i by one. Processing returns to step S4502.

Note that, in the flowchart in FIG. 45, in the case where two or more action sets and so on having the same starting (initial) piece of action information are accumulated, it is preferable to retain only the action set and so on containing the maximum number of pieces of action information and delete the others.

In the flowchart in FIG. 45, the termination condition of the loop of the action set acquisition processing from step S4505 to step S4510 is that the maximum number (counter j) of pieces of action information constituting the action set is greater than n₂. However, in the flowchart in FIG. 45, the termination condition of the loop of the action set acquisition processing from step S4505 to step S4510 may also be that a specific piece of action information (for example, "sleep" or "waking up") is included in the action set.

In addition, the set determination unit 731 may acquire an action set containing two or more pieces of action information from two or more pieces of action information between a first specific piece of action information and a second specific piece of action information. The pieces of action information from the first specific piece of action information to the second specific piece of action information constitute a segment that is a group of series of pieces of action information. Note that the segment may or may not include the first and second specific pieces of action information. For example, the first specific piece of action information is "waking up", and the second specific piece of action information is "commuting". Alternatively, for example, the first specific piece of action information is "commuting", and the second specific piece of action information is "returning home". Alternatively, the first specific piece of action information is "returning home", and the second specific piece of action information is "sleeping".

Next, an example of the recommendation acquisition processing in step S4404 will be described with reference to the flowchart in FIG. 46.

(Step S4601) The recommendation acquisition unit 733 substitutes 1 for a counter i.

(Step S4602) The recommendation acquisition unit 733 judges whether or not an i^{th} action set accumulated in step S4403 is present. If the i^{th} action set is present, processing proceeds to step S4603, and otherwise processing returns to the higher-level processing.

(Step S4603) The recommendation acquisition unit 733 acquires, from the i^{th} action set, keystone habit information including the piece of action information serving as a starting point. Keystone habit information includes action information. It is preferable that the keystone habit information includes one or more kinds of information among a start time, a time slot, and emotion information.

(Step S4604) The recommendation acquisition unit 733 acquires a recommendation template from the storage unit 71. For example, the recommendation acquisition unit 733 acquires, from the storage unit 71, a recommendation template that is paired with the emotion information contained in the keystone habit information acquired in step S4603.

(Step S4605) The recommendation acquisition unit 733 places the keystone habit information into the recommendation template acquired in step S4604 and forms a recommendation.

(Step S4606) The recommendation acquisition unit 733 places the recommendation formed in step S4605 into information to be transmitted.

(Step S4607) The recommendation acquisition unit 733 increments the counter i by one. Processing returns to step S4602.

Hereinafter, a specific operation example of an information system F according to the present embodiment will be described.

Now, it is assumed that the tables shown in FIGS. 33 to 35 are stored in the action management unit 313 of the information processing apparatus 7. In

FIGS. 34 and 35, it is assumed that all action-confirmation flags and all emotion confirmation flags are "1" (confirmed).

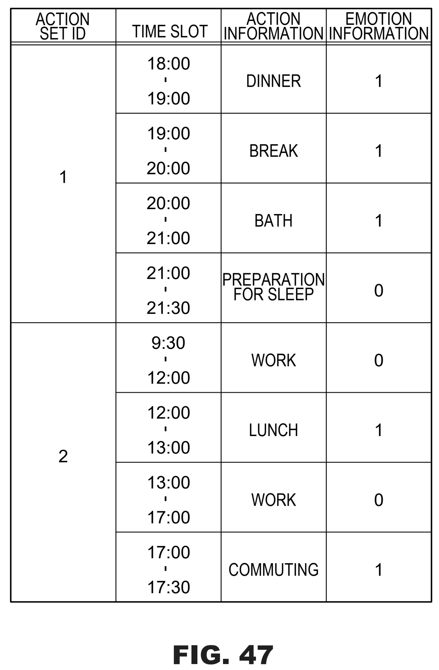

It is also assumed that, as a result of the processing from steps S4402 to S4404 described above, a group of keystone habit information corresponding to the user identifier "U" is stored based on the table in FIG. 35. This group of keystone habit information corresponds to the keystone habit management table shown in FIG. 47. The keystone habit management table contains records containing "action set ID," "time slot," "action information," and "emotion information".

Here, it is assumed that the user U of the terminal apparatus 6 wants to "wake up comfortably at 7 a.m." and inputs, into the terminal apparatus 6, the query "<time> 7:00 <action information> waking up <emotion information> 1". Note that emotion information "1" represents "positive" and "0" represents "negative".

Next, the terminal acceptance unit 62 of the terminal apparatus 6 accepts the query "<time> 7:00 <action information> waking up <emotion information> 1". Next, the terminal apparatus 6 transmits the query, paired with the user identifier "U," to the information processing apparatus 7.

Next, the query acceptance unit 721 of the information processing apparatus 7 receives the query "<time> 7:00 <action information> waking up <emotion information> 1" paired with the user identifier "U".

Next, the keystone habit acquisition unit 732 acquires the start time "7:00" included in the query. Then, the keystone habit acquisition unit 732 acquires, from the keystone habit management table (FIG. 47), the keystone habit information "<time slot> 18:00-19:00 <action information> dinner <emotion information> 1", which is action information and so on paired with the user identifier "U" and serving as the starting point in the action set corresponding to the time slot that precedes and is closest to the start time "7:00" (the records with "ID = 1" in FIG. 47).

Next, the processing unit 73 forms information to be output, which includes the acquired keystone habit information "<time slot> 18:00-19:00 <action information> dinner <emotion information> 1".

Next, the keystone habit output unit 741 transmits the formed information to the terminal apparatus 6.

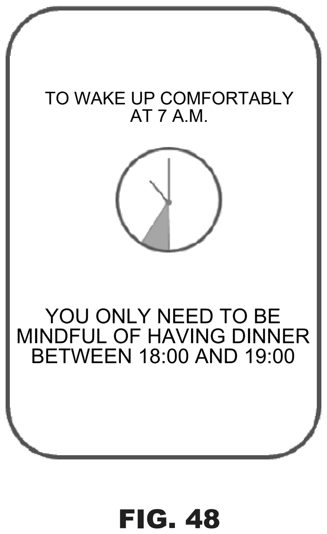

Next, the terminal apparatus 6 receives and outputs the keystone habit information and so on. An example of such output is shown in FIG. 48.

As described above, according to the present embodiment, the user's keystone habit can be presented.

According to the present embodiment, the user's keystone habit and the start time and the like thereof can be presented.

According to the present embodiment, a keystone habit corresponding to a query from the user can be presented to the user.

According to the present embodiment, a recommendation using a keystone habit can be provided.

Furthermore, according to the present embodiment, a recommendation using keystone habit and emotion information can be provided.

Note that the software that realizes the information processing apparatus 7 according to the present embodiment is the program described below. That is to say, this program is a program that enables a computer to function as: a set determination unit configured to reference an action management unit in which pieces of action information specifying a user's actions are stored in association with four or more time slots, and to determine an action set that is a series of two or more actions and satisfies a set condition; a keystone habit acquisition unit configured to acquire keystone habit information including a piece of action information specifying an action serving as a starting point of the action set determined by the set determination unit; and a keystone habit output unit configured to output the keystone habit information.

### Sixth Embodiment

In the present embodiment, a difference from the fifth embodiment is that the information processing apparatus is a terminal.

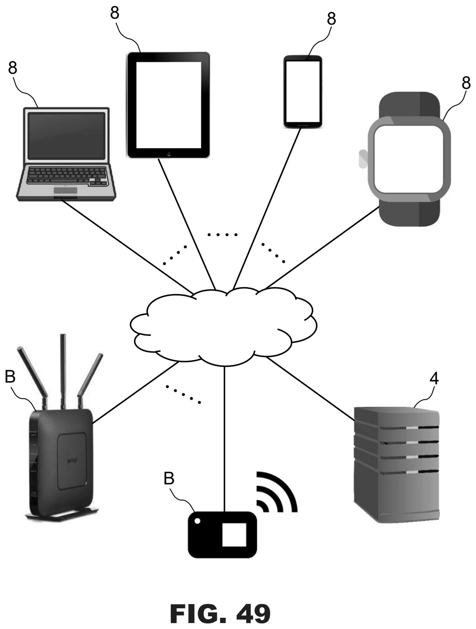

FIG. 49 is a conceptual diagram of an information system G according to the present embodiment. The information system G includes one or more information processing apparatuses 8, the server apparatus 4, and three or more communication apparatuses B.

Each information processing apparatus 8 is a terminal that determines an action set and acquires and outputs keystone habit information. Each information processing apparatus 8 is, for example, a smartphone, a tablet terminal, a smartwatch, or a so-called personal computer, and there is no limitation on the type thereof.

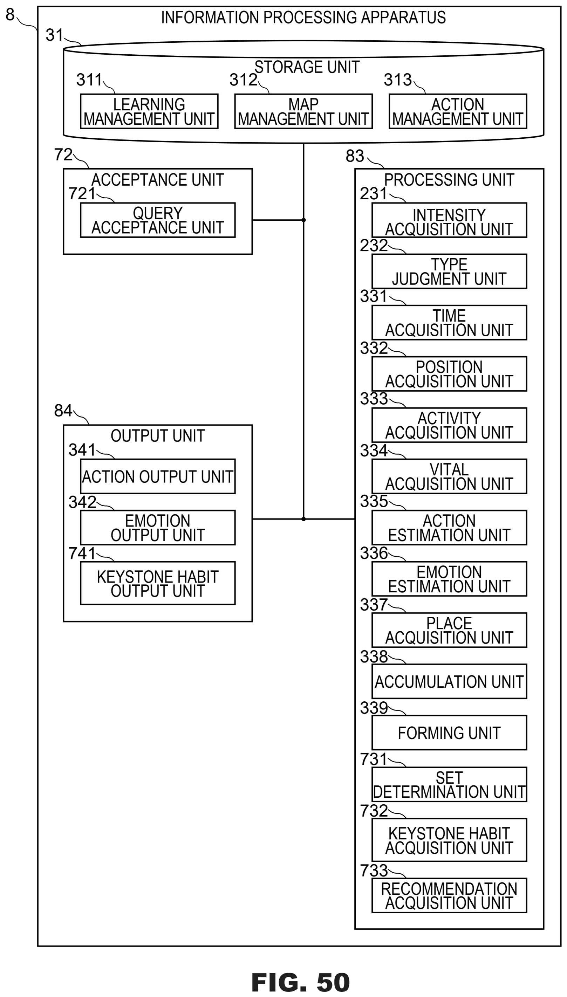

FIG. 50 is a block diagram of the information system G according to the present embodiment. Each information processing apparatus 8 includes the storage unit 31, the acceptance unit 72, a processing unit 83, and an output unit 84. The storage unit 31 includes the learning management unit 311, the map management unit 312, and the action management unit 313. The acceptance unit 72 includes the query acceptance unit 721. The processing unit 83 includes the intensity acquisition unit 231, the type judgment unit 232, the time acquisition unit 331, the position acquisition unit 332, the activity acquisition unit 333, the vital acquisition unit 334, the action estimation unit 335, the emotion estimation unit 336, the place acquisition unit 337, the accumulation unit 338, the forming unit 339, the set determination unit 731, the keystone habit acquisition unit 732, and the recommendation acquisition unit 733. The output unit 84 includes the action output unit 341, the emotion output unit 342, and the keystone habit output unit 741.

Next, the operation example of the information processing apparatus 8 is the same as the processing described with reference to the flowchart in FIG. 44. Note that the acceptance unit 72 and the query acceptance unit 721 of the information processing apparatus 8 typically accepts input from a user.

It is preferable that the information processing apparatus 8 have the functions of the terminal apparatus 2 and the action acquisition apparatus 3.

As described above, according to the present embodiment, the user's keystone habit can be presented.

According to the present embodiment, the user's keystone habit and the start time and the like thereof can be presented.

According to the present embodiment, an action set can be determined by using emotion information.

According to the present embodiment, a keystone habit corresponding to a query from the user can be presented to the user.

According to the present embodiment, a recommendation using a keystone habit can be provided.

Furthermore, according to the present embodiment, a recommendation using keystone habit and emotion information can be provided.

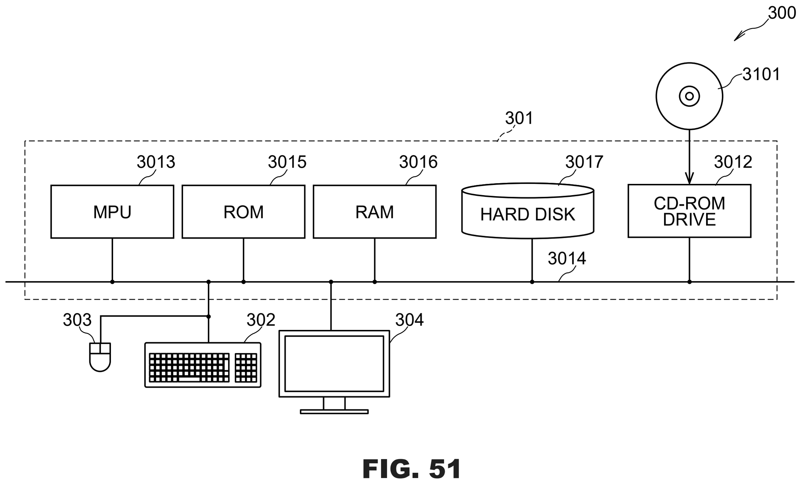

The apparatuses indicated by the reference numeral 7 in FIG. 42 and the reference numerals 8 in FIG. 49 each represent, in the form of an external view, a computer that executes the programs described in this specification to realize the information processing apparatuses and the like according to the various embodiments described above. The above-described embodiments can be realized using computer hardware and a computer program executed thereon. The apparatuses indicated by the reference numeral 7 in FIG. 42 and the reference numeral 8 in FIG. 49 each represent this computer system 300 in the form of a schematic diagram, and FIG. 51 is a block diagram of the system 300.

As exemplified by the apparatuses indicated by the numeral 7 in FIG. 42 and the numeral 8 in FIG. 49, the computer system 300 includes a computer 301 that includes a CD-ROM drive, a keyboard 302, a mouse 303, and a monitor 304.

In FIG. 51, the computer 301 includes, in addition to the CD-ROM drive 3012, an MPU 3013, a bus 3014 that is connected to the CD-ROM drive 3012 and so on, a ROM 3015 for storing programs such as a boot-up program, a RAM 3016 that is connected to the MPU 3013 and is used to temporarily store application program instructions and provide a temporary storage space, and a hard disk 3017 for storing application programs, system programs, and data. Here, although not shown in the figure, the computer 301 may further include a network card that provides connection to a LAN.

The program that enables the computer system 300 to perform the functions of the information processing apparatus 7 and so on according to the above-described embodiments may be stored in the CD-ROM 3101, inserted into the CD-ROM drive 3012, and furthermore transferred to the hard disk 3017. Alternatively, the program may be transmitted to the computer 301 via a network (not shown) and stored on the hard disk 3017. The program is loaded into the RAM 3016 when the program is to be executed. The program may be directly loaded from the CD-ROM 3101 or the network.

The program does not necessarily have to include an operating system (OS), a third party program, or the like that enables the computer 301 to perform the functions of the information processing apparatus 7 and so on according to the embodiments described above. The program need only contain the part of the instruction that calls an appropriate function (module) in a controlled manner to achieve a desired result. How the computer system 300 works is well known and the detailed descriptions thereof will be omitted.

In the above-described program, the step of transmitting information, the step of receiving information and so on do not include processing performed by hardware, for example, processing performed by a modem or an interface card in the step of transmitting (processing that can only be performed by hardware).

There may be a single or multiple computers executing the above-described program. That is to say, centralized processing or distributed processing may be performed.

Also, as a matter of course, in each of the above-described embodiments, two or more communication means that are present in one apparatus may be physically realized using one medium.

Also, in the above-described embodiments, each kind of processing may be realized as centralized processing that is performed by a single apparatus, or distributed processing that is performed by multiple apparatuses.

As a matter of course, the present invention is not limited to the above-described embodiments, and various changes are possible, and such variations are also included within the scope of the present invention.

### Industrial Applicability

As described above, the information processing apparatus according to the present invention has the effect of being able to present a user's keystone habit, and is useful as an information processing apparatus or the like.

## Claims

1. An information processing apparatus comprising:
a set determination unit configured to reference an action management unit in which pieces of action information specifying a user's actions are stored in association with four or more time slots, to acquire the number of occurrences of an action set that is a series of two or more pieces of action information that are temporally continuous, and to determine an action set whose number of occurrences satisfies a set condition on the number of occurrences of the action set;
a keystone habit acquisition unit configured to acquire keystone habit information including a piece of action information that corresponds to an initial time slot among the series of two or more pieces of action information in the action set determined by the set determination unit and serves as a starting point; and
a keystone habit output unit configured to output the keystone habit information.

2. The information processing apparatus according to claim 1,
wherein the set condition is that the number of occurrences of the action set among the pieces of action information in the action management unit is not less than N, where N is a natural number not less than 2.

3. The information processing apparatus according to claim 1,
wherein the keystone habit acquisition unit acquires keystone habit information that includes a piece of action information specifying an action serving as a starting point of the action set and a start time included in a time slot corresponding to the piece of action information.

4. The information processing apparatus according to any one of claims 1 to 3, further comprising:
a time acquisition unit configured to acquire a time;
a position acquisition unit configured to acquire position information corresponding to the time; and
an action estimation unit configured to, using two or more pieces of action source information including the position information corresponding to the time, acquire a piece of action information specifying an action of the user during a time slot specified by the time contained in the two or more pieces of action source information,
wherein at least one of the two or more pieces of action information corresponding to time slots in the action management unit is the piece of action information corresponding to the time slot acquired by the action estimation unit.

5. The information processing apparatus according to any one of claims 1 to 3,
wherein the pieces of action information in the action management unit are associated with time slots and pieces of emotion information specifying emotions,
the information processing apparatus further comprises a query acceptance unit configured to accept a query including a time, a piece of action information, and a piece of emotion information, and
the keystone habit acquisition unit acquires keystone habit information including a piece of action information that corresponds to the time slot including the time included in the query and the piece of emotion information included in the query, and serves as a starting point of the action set including the piece of action information included in the query.

6. The information processing apparatus according to claim 1, further comprising:
a query acceptance unit configured to accept a query including a time and a piece of action information, and
the keystone habit acquisition unit determines a piece of action information specifying an action serving as a starting point of the action set determined by the set determination unit, and a time slot corresponding to the time and the piece of action information contained in the query, and acquires keystone habit information including a piece of action information specifying an action serving as a starting point of an action set corresponding to a time slot that precedes the determined time slot and is closest to the determined time slot.

7. The information processing apparatus according to claim 1,
wherein the keystone habit output unit outputs a recommendation including the keystone habit information.

8. The information processing apparatus according to claim 6,
wherein the pieces of action information in the action management unit are associated with pieces of emotion information specifying emotions,
the information processing apparatus further comprises a recommendation acquisition unit configured to acquire a piece of emotion information corresponding to the piece of action information included in the keystone habit information or the piece of action information contained in the query, and to form a recommendation using the piece of emotion information and the keystone habit information, and
the keystone habit output unit outputs the recommendation.

9. An information processing method that is realized by an information processing apparatus including a set determination unit, a keystone habit acquisition unit, and a keystone habit acquisition unit output unit, comprising:
a set determination step in which the set determination unit references an action management unit in which pieces of action information specifying a user's actions are stored in association with four or more time slots, acquires the number of occurrences of an action set that is a series of two or more pieces of action information that are temporally continuous, and determines an action set whose number of occurrences satisfies a set condition on the number of occurrences of the action set;
a keystone habit acquisition step in which the keystone habit acquisition unit acquires keystone habit information including a piece of action information that corresponds to an initial time slot among the series of two or more pieces of action information in the action set determined by the set determination unit and serves as a starting point; and
a keystone habit output step in which the keystone habit output unit outputs the keystone habit information.

10. A recording medium having recorded thereon a program for enabling a computer to function as:
a set determination unit configured to reference an action management unit in which pieces of action information specifying a user's actions are stored in association with four or more time slots, to acquire the number of occurrences of an action set that is a series of two or more pieces of action information that are temporally continuous, and to determine an action set whose number of occurrences satisfies a set condition on the number of occurrences of the action set;
a keystone habit acquisition unit configured to acquire keystone habit information including a piece of action information that corresponds to an initial time slot among the series of two or more pieces of action information in the action set determined by the set determination unit and serves as a starting point; and
a keystone habit output unit configured to output the keystone habit information.
